(19) Europäisches Patentamt
European Patent Office
Office européen des brevets

(11) EP 2 216 052 A1

(12) EUROPEAN PATENT APPLICATION
published in accordance with Art. 153(4) EPC

(43) Date of publication:
**11.08.2010 Bulletin 2010/32**

(21) Application number: **08845375.8**

(22) Date of filing: **28.10.2008**

(51) Int Cl.:
***A61K 51/00*** *(2006.01)* ***C07D 471/04*** *(2006.01)*

(86) International application number:
**PCT/JP2008/069503**

(87) International publication number:
**WO 2009/057577 (07.05.2009 Gazette 2009/19)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **30.10.2007 JP 2007282363**

(71) Applicant: **Nihon Medi-Physics Co., Ltd.**
**Tokyo 136-0075 (JP)**

(72) Inventors:
- **TANIFUJI, Shigeyuki**
**Sodegaura-shi**
**Chiba 299-0266 (JP)**
- **NAKAMURA, Daisaku**
**Sodegaura-shi**
**Chiba 299-0266 (JP)**
- **TAKASAKI, Shinya**
**Sodegaura-shi**
**Chiba 299-0266 (JP)**
- **OKUMURA, Yuki**
**Sodegaura-shi**
**Chiba 299-0266 (JP)**

(74) Representative: **Wilhelms · Kilian & Partner**
**Patentanwälte**
**Eduard-Schmid-Straße 2**
**81541 München (DE)**

# (54) USE OF NOVEL COMPOUND HAVING AFFINITY FOR AMYLOID, AND PROCESS FOR PRODUCTION OF THE SAME

(57) The invention provides a reagent which can detect amyloid *in vitro* and *in vivo* with high sensitivity using a compound which has affinity with amyloid.
The reagent for detecting amyloid deposited in a biological tissue comprises the compound represented by the following formula (1) or a salt thereof:

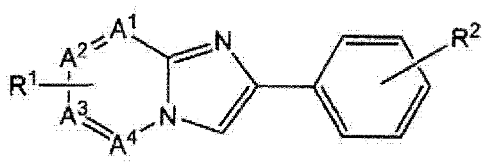

(1)

wherein $A^1$, $A^2$, $A^3$ and $A^4$ independently represent a carbon or nitrogen,
$R^1$ is a group selected from the group consisting of a hydrogen, hydroxyl group, carboxyl group, sulfuric acid group, amino group, nitro group, cyano group, non-radioactive halogen substituent, alkyl substituent with 1 to 4 carbon atoms and alkoxy substituent, with 1 to 4 carbon atoms, and
$R^2$ is a radioactive halogen substituent,
provided that at least one of $A^1$, $A^2$, $A^3$ and $A^4$ represents a carbon, and $R^1$ binds to a carbon represented by $A^1$, $A^2$, $A^3$ or $A^4$.

EP 2 216 052 A1

## Description

TECHNICAL FIELD

The present invention relates to use and process for production of a novel compound having affinity for amyloid, and especially relates to a reagent for detecting amyloid in biological tissues, which is useful for detection of amyloid at lesion sites in diagnosis of systemic amyloidosis diseases and other diseases with amyloid accumulation.

BACKGORUND ART

Diseases with the onset of deposition of a fibrous protein called amyloid in various organs or tissues in bodies are generally referred to as amyloidosis. A feature common to amyloidosis is that the fibrous protein called amyloid which is enriched with the β-sheet structure is deposited at various organs systemically or at sites topically so that functional abnormalities are triggered in the organs or tissues. Amyloid generally refers to protein aggregates formed by aggregation of various amyloid precursor proteins such as amyloidβ, mutant transthyretin and β2-microglobulin in a living body. The amyloid has a characteristic structure enriched with β-sheet seven if it is formed of any of the amyloid precursor proteins. Thus, compounds such as Congo-red and Thioflavin T capable of binding to β-sheet are characteristic in that they have affinity for amyloid.

Amyloidosis is classified into a systemic amyloidosis and a localized amyloidosis depending upon amyloid deposition patterns.

The systemic amyloidosis is a disease in which amyloid deposition occurs at various parts of whole body. Examples of the systemic amyloidosis include familial amyloidosis in which amyloid produced in the liver is deposited in organs throughout the whole body so as to cause disorder, senile TTR amyloidosis in which amyloid is deposited in heart and a large joint such as hand joint, dialysis amyloidosis occurring at sites such as bones and jontns of long-term dialysis patients, reactive AA amyloidosis (secondary amyloidosis) which is caused by deposition of amyloid derived from serum amyloid A which is an acute phase protein produced following a chronic inflammatory disease such as chronic rheumatism, and immunocytic amyloidosis in which amyloid derived from immunoglobulin is deposited in various organs throughout the whole body.

The localized amyloidosis is a disease in which amyloid deposition occurs only at some organs. Examples of the localized amyloidosis includes brain amyloidosis such as Alzheimer's disease in which amyloid is deposited in brain, cerebrovascualr amyloidosis and Creutzfeldt Jakob disease, endocrine amyloidosis in which amyloid is deposited in pancreatic inslet accompanied by type II diabetes and insulinoma or deposited in heart atrium, cutaneous amyloidosis in which amyloid is deposited in skin and localized nodular amyloidosis in which nodular amyloid is deposited in skin and lungs.

Diagnosis of amyloidosis is, in case of the systemic amyloidosis, at first conducted by collecting a tissue from a region where biopsy is available such as skin, kidney and gastrointestinal tract, and staining it with Congo-red or Thioflavin T. congo-red is a fluorescent compound high in affinity to β-sheet structure of amyloid, and since Congo-red shows doubly refraction under polarization microscope due to its orientation, it can selectively stain amyloid deposition in tissues. Similarly, Thioflavin T is also a fluorescent compound having affinity with amyloid, and used similarly to Congo-red. After the tissue staining is found to be positive, definite diagnosis is conducted by means of immunostaining with an antibody or the like in combination therewith. However, positive diagnosis is often difficult by staining with Congo-red and Thioflavin T even under polarization microscopy.

On the other hand, imaging diagnosis of the systemic amyloidosis has been considered with recent wide spread of image diagnosis device such as PET, SPECT and MRI.

However, when Congo-red and Thioflavin T are labeled and used as probes for imaging diagnosis, they are problematic in that binding specificity to amyloid is inferior so that good detection sensitivity cannot be obtained.

Further, since congo-red has carcinogenicity, it cannot be sued for diagnosis of human body.

Thus, it has been proposed bis-(3-hydroxycarbonyl-4-hydroxy)styrylbenzene (BSB) as a congo-red derivative and derivatives thereof have been proposed as a fluorescent reagent for detecting amyloid which is high in affinity and detection sensitivity for systemic amyloid and can be used for *in vivo* detection (non-Patent Document 14, Patent Document 7).

It has been reported that BSB is high in affinity for amyloid caused by brain amyloidosis and systemic amyloidosis, and has no benzizine structure in its structure, and thus it has little carcinogenic problem and can be radioactive-labeled for use as a probe for imaging diagnosis.

On the other hand, for Alzheimer's disease (hereinafter, referred to as AD) which is a typical brain amyloidosis, attempts have already been made to detect AD *in vivo* using a compound having high affinity with amyloid as a marker since it is impossible to collect a biopsy. Many of such probes for imaging diagnoses of cerebral amyloid are hydrophobic low-molecular weight compounds that are high in affinity with amyloid and high in cerebral transferability and are labeled with various radioactive species such as $^{11}C$, $^{18}F$ and $^{123}I$. For Example, reports tell $^{11}C$ or radioactive halogen labeled forms of compounds including various thioflavin derivatives such as 6-indo-2-[4 -(N,N-dimethylamino) phenyl]benzothiazole (hereinafter referred to as TZDM) and 6-hydroxy-2-[4 -(N-methylamino)phenyl]benzothiazole (hereinafter referred to as 6-OH-BTA-1) (Patent Document 1, Non-Patent Document 3); stilbene compounds such as (E)-4-methylamino-4 -hydroxystilbene ((hereinafter referred to as SB-13) and (E)-4-dimethylamino-4 -iodostilbene (hereinafter referred to as m-I-SB) (Patent Document 2, Non-Patent Document 4, Non-Patent Document 5); benzoxazole derivatives such as 6-iodo-2-[4-(N,N-dimethylamino)phenyl]benzoxazole (hereinafter referred to as IBOX) and 6-[2-(fluoro)ethoxy]-2-2-(2-dimethylaminothiazol-5-yl)ethenyl]benzoxazole (Non-Patent Document 6, Non-Patent Document 7), DDNP derivatives such as 2-(1-{6-[(2-fluoroethyl)(methyl)amino]-2-naphthyl}ethylidene)malononitrile (hereinafter referred to as FDDNP) (Parent Document 4, Non-Patent Document 8); and imidazopyridine derivatives such as 6-iodo-2-[4 - (N,N-dimethylamino)phenyl]imidazo[1,2-a]pyridine (hereinafter referred to as IMPY) (Patent Document 3, Non-Patent Document 9). Further, some of these probes for imaging diagnosis have been studied on human imaging and have been reported to show a significant radioactivity accumulation in AD patient's brain compared with normal persons (Non-Patent Document 10, Non-Patent Document 11, Non-Patent Document 12, and Non-patent Document 13).
International Publication No. WO2007/002540 pamphlet discloses a series of compounds with a group having affinity with amyloid, to which a radioisotope labeling site is attached via ethylene glycol or polyethylene glycol (Patent Document 5).
International Publication No. WO2007/063946 pamphlet discloses a series of compounds to which a five-membered aromatic heterocyclic group is attached in order to prevent them from being metabolized in brain (Patent Document 6).
[Patent Document 1] JP-T-2004-506723
[Patent Document 2] JP-T-2005-504055
[Patent Document 3] JP-T-2005-512945
[Patent Document 4] JP-T-2002-523383
[Patent Document 5] International Publication No. WO2007/002540 pamphlet
[Patent Document 6] International Publication No. WO2007/063946 pamphlet
[Patent Document 7] International Publication No. WO2005/016888 pamphlet
[Non-Patent Document 1] J. A. Hardy & G. A. Higgins, "Alzheimer's Disease: The Amyloid Cascade Hypotheses.", Science, 1992, 256, p.184-185
[Non-Patent Document 2] G. McKhann et al., clinical diagnosis of Alzheimer's disease: Retort of the NINCDS-ADRDA Work Group under the auspices of Department of Health and Human Services Task Force on Alzheimer's Disease.", Necrology, 1984, 34, p.939-944
[Non-Patent Document 3] Z.-P. Zhuang et al., "Radioiodinated Styrylbenzenes and Thioflavins as Probes for Amyloid Aggregates.", J. Med. Chum., 2001, 44, p.1905-1914
[Non-Patent Document 4] Masahiro One et al., "11C-labeled stilbene derivatives as Aβ-aggregate-specific PET imaging agents for Alzheimer's disease.", Nuclear Medicine and Biology, 2003, 30, p.565-571
[Non-Patent Document 5] H. F. Kung et al., "Novel Stilbenes as Probes for amyloid plaques.", J. American Chemical Society, 2001, 123, p.12740-12741
[Non-Patent Document 6] Zhi-Ping Zhuang et al., "IBOX(2-(4 -dimethylaminophenyl)-6-iodobensoxazole) : a ligand for imaging amyloid plaques in the brain.", Nuclear medicine and Biology, 2001, 28, p.887-894
[Non-Patent Document 7] Furumoto Y et al., "[11C]BF-227: A New 11C-Labeled 2-Ethenylbenzoxazole Derivative for Amyloid-β Plaques Imaging.", European Journal of Nuclear Medicine and Molecular Imaging, 2005, 32, Sup.1, P759
[Non-Patent Document 8] Eric D. Agdeppa et al., "2-Dialkylamino-6-Acylmalononitrile Substituted Naphthalenes (DDNP Analogs): Novel Diagnostic and Therapeatic Tools in Alzheimer's Disease.", Molecular Imaging and Biology, 2003, 5, p.404-417
[Non-Patent Document 9] Zhi-Ping Zhuang et al., "Structure-Activity. Relationship of Imidazo[1,2-a]pyridines as Ligands for Detecting β-Amyloid Plaques in the Brain.", J. Med. Chem, 2003, 46, p.237-243
[Non-Patent Document 10] W. E. Klunk et al., "Imaging brain amyloid in Alzheimer's disease with Pittsburgh Compound-B.", Ann. Neurol., 2004, 55, p.306-319
[Non-Patent Document 11] Nicolaas P. L. G. Verhoeff et al., "In-Vivo Imaging of Alzheimer Disease β-Amyloid with [11C]SB-13 PET.", American Journal of Geriatric Psychiatry, 2004, 12, p.584-595
[Non-Patent Document 12] Hiroyuki Arai et al., "[11C]-BF-227 AND PET to visualize Amyloid in Alzheimer's Disease Patients", Alzheimer's & Dementia: The Journal of the Alzheimer's Association, 2006, 2, sup. 1, S312
[Non-Patent Document 13] Christopher M. Clark et al., "Imaging Amyloid with I123 IMPY SPECT", Alzheimer's & Dementia: The Journal of the Alzheimer's Association, 2006, 2, Sup. 1, S342
[Non-Patent Document 14] D. M. Skovronsky et al., Proc. Natl. Acad. Sci., 2000, 97, 7609

## DISCLOSURE OF THE INVENTION

## PROBLEMS TO BE SOLVED BY THE INVENTION

As described above, various compounds, are disclosed as probes for imaging diagnosis for amyloid, and researched for clinical application.
Experiments in normal mice show that [$^{125}$I]-labeled TZDM, IBOX and m-I-SB are all transferred into brain 2 minutes after administration. However, these compounds are insufficient in clearance from normal tissues, and tend to accumulate gradually in brain as time passes after administration (JP-T-2005-512945; Zhi-Ping Zhuang et al., Nuclear Medicine and Biology, 2001, 28, p.887-894; H. F. Kung et al., J. Am. Chem. Soc., 2001, 123, p.12740-12741). When the clearance from normal tissues is insufficient, a problem arises in that sufficient contrast cannot be obtained at amyloid accumulation sites. [$^{11}$C]-labeled SB-13 shows a clearance from normal tissues in experiments in rats, however, it cannot be said that the clearance is sufficiently fast (Masahiro Onto et al., Nuclear Medicine and Biology, 2003, 30, p.565-571).
Meanwhile, it is revealed that compounds having an imidazopyridine skeleton such as IMPY have a property of transferring to brain and accumulating at amyloid after administration, and also have an excellent property of rapid clearance from normal tissues unlike the above-described compounds, as a result of experiments using [$^{125}$I]-labeled compounds. However, IMPY is a compound, positive in reverse mutation test. In order to use this compound as a probe for imaging diagnosis, sufficient care must be taken about dosage and administration manner (International Publication No. WO03/106439 pamphlet).
FDDNP is also reported to be positive in reverse mutation test (International Publication No. WO03/106439 pamphlet).
The present invention has been made under such circumstances where various compounds as probes targeting amyloid have been disclosed but there has been no compounds which is confirmed to have a clinically tolerable property, and aims at making it possible to detect amyloid *in vivo* or *in vitro* with high sensitivity by providing a novel compound having affinity with amyloid.

## MEANS FOR SOLVING THE PROBLEM

The present inventors have found that a specific novel compound with an imidazopyridine-phenyl skeleton has affinity with amyloid, and amyloid can be detested in vivo or in vitro with high sensitivity by use of a series of the compound as a probe. Thus, the present invention has been completed.
That is, according to one aspect of the present invention, a compound represented by the following formula (1) :
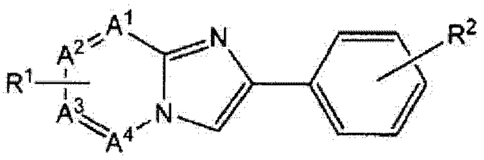

(1)

or a salt thereof, and a reagent for detecting amyloid deposited in a biological tissue which comprises a compound represented by the formula (1) or a salt thereof are provided.
The biological tissue can be various tissues at which amyloid is known to deposit in amyloidosis. Typical examples of such biological tissues include brain, heart, lung, pancreas, bone and joint, and as the most typical biological tissue, mention may be made of brain. The typical amyloidosis in case of brain include Alzheimer's disease and dementia with Lewy bodies.
In the formula (1), $A^1$, $A^2$, $A^3$ and $A^4$ independently represent a carbon or nitrogen, and it is necessary that at least one of these represents a carbon. Preferably, three or more of $A^1$, $A^2$, $A^3$ and $A^4$ represent carbons, and more preferably, all of them represent carbons. In the formula (1), $R^1$ binds to a carbon represented by $A^1$, $A^2$, $A^3$ or $A^4$. Also, a binding site for $R^1$ is preferably a carbon represented by $A^3$, that is, a carbon at 6-position.
In the formula (1), $R^1$ may be a group selected from the group consisting of a hydrogen, hydroxyl group, carboxyl group, sulfuric acid group, amino group, nitro group, cyano group, non-radioactive halogen, substituent, alkyl substituent with 1 to 4 carbon atoms and alkoxy substituent with I to 4 carbon atoms. $R^1$ is preferably a hydroxyl group, alkyl substituent with 1 to 4 carbon atoms or alkoxy substituent with 1 to 4 carbon atoms, and more preferably a hydroxyl group, methyl substituent or methoxy substituent.
$R^2$ may be a radioactive halogen substituent, and preferably a radioactive halogen substituent selected from the group consisting of $^{18}$F, $^{75}$Br, $^{76}$Br, $^{123}$I, $^{124}$I, and $^{131}$I, and more preferably a halogen selected from $^{18}$F, $^{76}$Br, $^{123}$I

and $^{125}I$.

That is, according to one aspect of the present invention, a compound represented by the following formula (2):

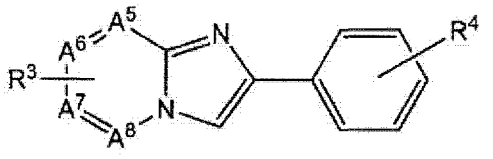

(2)

or a salt thereof, and a reagent for detecting amyloid deposited in a biological tissue which comprises a compounds represented by the formula (2) or a salt thereof are provided, The biological tissue includes those described about a compound of the formula (1).

In the formula (2), $A^5$, $A^6$, $A^7$ and $A^8$ independently represent a carbon or nitrogen, and it is necessary that at least one of these represents a carbon. Preferably, three or more of $A^5$, $A^6$, $A^7$ and $A^8$ represent carbons, and more preferably, all of them represent carbons. In the formula (2), $R^3$ binds to a carbon represented by $A^5$, $A^6$, $A^7$ or $A^8$. Also, a binding site for $R^3$ is preferably a carbon represented by $A^7$, that is, a carbon at 6-position.

In the formula (2), $R^3$ may be a radioactive halogen substituent, preferably a radioactive halogen substituent group selected from the group consisting of $^{18}F$, $^{75}Br$, $^{76}Br$, $^{123}I$, $^{124}I$, $^{125}I$ and $^{131}I$, and more preferably a radioactive halogen substituent, selected from the group consisting of $^{18}F$, $^{76}Br$ $^{123}I$ and $^{125}I$.

$R^4$ may be a group selected from the group consisting of a hydrogen, carboxyl group, sulfuric acid group, nitro group, cyano group, non-radioactive halogen substituent, alkyl substituent with 1 to 4 carbon atoms and methoxy substituent. $R^4$ is preferable an alkyl substituent with 1 to 4 carbon atoms or methoxy substituent, and more preferably methyl substituent or methoxy substituent.

The above compound of the formulae (1) is a novel compound, and according to still another aspect of the present invention, a process for production of a radioactive halogen-labeled organic compound is provided, which comprises a step of preparing a reaction solution containing, together with a radioactive halogen ion, a compound represented by the following formula (3):

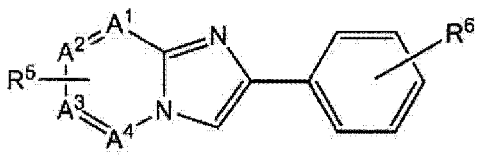

(3)

wherein, $A^1$, $A^2$, $A^3$ and $A^4$ independently represent a carbon or nitrogen,
$R^5$ is a group selected from the group consisting of a hydrogen, hydroxyl group, carboxyl group, sulfuric acid group, amino group, nitro group, cyano group, non-radioactive halogen substituent, alkyl substituent with 1 to 4 carbon atoms and alkoxy substituent with 1 to 4 carbon atoms,
$R^6$ is a group selected from the group consisting of a non-radioactive halogen substituent, nitro substituent, trialkylstannyl substituent having alkyl chains with 1 to 4 carbon atoms, triphenylstannyl group and trialkylammonium group having alkyl chains with 1 to 4 carbon atoms,
provided that at least one of $A^1$, $A^2$, $A^3$ and $A^4$ represents a carbon, and $R^5$ binds to a carbon represented by $A^1$, $A^2$, $A^3$ or $A^4$,

or a salt thereof,
and a step of giving a reaction condition to the reaction solution to synthesize a compound represented by the following formula (1):

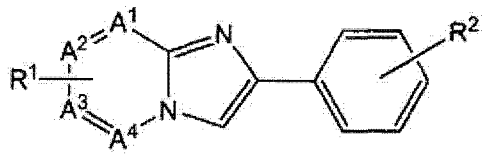

(1)

wherein, $A^1$, $A^2$, $A^3$ and $A^4$ independently represent a carbon or nitrogen,
$R^1$ is a group selected from the group consisting of a hydrogen, hydroxy group, carboxyl group, sulfuric acid group, amino group, nitro group, cyano group, non-radioactive halogen substituent and alkyl substituent with 1 to 4 carbon atoms and alkoxy substituent with 1 to 4 carbon atoms,
$R^2$ is a radioactive halogen substituent,
provided that at least one of $A^1$, $A^2$, $A^3$ and $A^4$ represents a carbon, and $R^1$ binds to a carbon represented by $A^1$, $A^2$, $A^3$ or $A^4$,

or a salt thereof.

In the formula (3), $A^1$, $A^2$, $A^3$ and $A^4$ independently represent a carbon or nitrogen, and it is necessary that at least one of these represents a carbon. Preferably, three or more of $A^1$, $A^2$, $A^3$ and $A^4$ represent carbons, and more preferably, all of them represent carbons. In the formula (3), $R^1$ binds to a carbon represented by $A^1$, $A^2$ $A^3$ or $A^4$. Also, a binding site for $R^1$ is preferably a carbon represented by $A^3$, that is, a carbon at 6-position.

In the formula (3), $R^5$ may be a group selected from the group consisting of a hydrogen, hydroxyl group, carboxyl group, sulfuric acid group, amino group, nitro group, cyano group, non-radioactive halogen substituent, alkyl substituent with 1 to 4 carbon atoms and alkoxy substituent with 1 to 4 carbon atoms. $R^5$ is preferably a hydroxyl group, alkyl substituent with 1 to 4 carbon atoms or alkoxy substituent with 1 to 4 carbon atoms and more preferably a hydroxyl group, methyl substituent or methoxy substituent.

$R^6$ may be a group selected from the group consisting of a non-radioactive halogen substituent, nitro group, trialkylstannyl substituent having alkyl chains with 1 to 4 carbon atoms, triphenylstannyl group and trialkylammonium group having alkyl chains with 1 to 4 carbon atoms.
As the non-radioactive halogen substituent, a halogen capable of being a target of nucleophilic substitution reaction using a radioactive fluorine or radioactive iodine, and preferable chlorine, iodine or bromine can be used.

The above compound of the formula (2) is a novel compound, and according to still another aspect of the present invention, a process for production of a radioactive halogen-labeled organic compound is provided, which comprises a step of preparing a reaction solution containing, together with a radioactive halogen ion, a compound represented by the following formula (4):

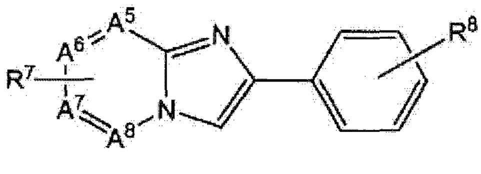

(4)

wherein $A^5$, $A^6$, $A^7$ and $A^8$ independently represent a carbon or nitrogen,
$R^7$ is a group selected from the group consisting of a non-radioactive halogen substituent, nitro group, trialkylstannyl substituent having alkyl chains with 1 to 4 carbon atoms, triphenylstannyl group and trialkylammonium group having alkyl chains with 1 to 4 carbon atoms, and
$R^8$ is a group selected from the group consisting of a hydrogen, carboxyl group, sulfuric acid group, nitro group, cyano group, non-radioactive halogen substituent, alkyl substituent with 1 to 4 carbon atoms and methoxy substituent,
provided that at least one of $A^5$, $A^6$, $A^7$and $A^8$ represents a carbon, and $R^7$ binds to a carbon represented by $A^5$, $A^6$, $A^7$ or $A^8$,

or a salt thereof,
and step of giving a reaction condition to the reaction solution to synthesize a compound represented by the following formula (2):

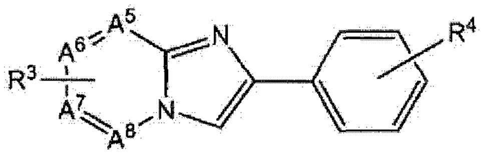

(2)

wherein $A^5$, $A^6$, $A^7$ and $A^8$ independently represent a carbon or nitrogen,
$R^3$ is a radioactive halogen substituent, and
$R^4$ is a group selected from the group consisting of a hydrogen, carboxyl group, sulfuric acid group, nitro group, cyano group, non-radioactive halogen substituent and alkyl substituent with 1 to 4 carbon atoms and methoxy substituent,
provided that at least one of $A^5$, $A^6$, $A^7$ and $A^8$ represents a carbon, and $R^3$ binds to a carbon represented by $A^5$, $A^6$, $A^7$ of $A^8$,

or a salt thereof.

In the formula (4), $A^5$, $A^6$, $A^7$ and $A^8$ independently represent a carbon or nitrogen, and it is necessary that at least one of these represents a carbon. Preferably, three or more of $A^5$, $A^6$, $A^7$ and $A^8$ represent carbons, and more preferably, all of them represent carbons. In the formula (4), $R^7$ binds to a carbon represented by $A^5$, $A^6$, $A^7$ or $A^8$. Also, a binding site for $R^8$ is preferably a carbon represented by $A^7$, that is, a carbon at 6-position.

In the formula (4), $R^7$ may be a group selected from the group consisting of a non-radioactive halogen substituent, nitro substituent, trialkylstannyl substituent having alkyl chains with 1 to 4 carbon atoms, triphenylstannyl group and trialkylammonium group having alkyl chains with 1 to 4 carbon atoms.
As the non-radioactive halogen substituent, a halogen capable of being a target of nucleophilic substitution reaction using a radioactive fluorine or radioactive iodine, and preferably chlorine, iodine or bromine can be used.

$R^8$ may be a group selected from the group consisting of a hydrogen, carboxyl group, sulfuric acid group, nitro group, cyano group, non-radioactive halogen substituent, alkyl substituent with 1 to 4 carbon atoms or methoxy substituent. $R^8$ is preferably an alkyl substituent with 1 to 4 carbon atoms or methoxy substituent and more preferably a methyl substituent or methoxy substituent.

The step of preparing a reaction solution comprising a precursor compound represented above in the formula (3) or (4) or a salt thereof and a radioactive halogen ion can be conducted, for example, by dissolving the precursor compound or a salt thereof in an inert organic solvent, and adding thereto a radioactive halogen ion-containing solution which has been obtained with a known method.
As the inert organic solvent, various solvents which do not have reactivity with the precursor compound or a salt thereof and the radioactive halogen ion can be used, for example, when a radioactive halogen ion to be used is a radioactive iodine, methanol can preferably be used, and when a radioactive halogen ion to be used is a radioactive fluorine, acetonitrile can preferably be used.
The reaction condition to be given for the reaction solution in the step of synthesizing a compound represented above of the formulae (1) or (2), or a salt thereof is not particularly limited as long as it is a conditions in which a reaction of substituting $R^6$ or $R^7$ of a compound of the formula (3) or (4) with the radioactive halogen ion added in the reaction solution is allowed to proceed, and a known reaction condition that fits kinds of the radioactive halogen ion can be used.

In the process for producing the radioactive halogen-labeled organic compound of the present invention, as the radioactive halogen ion, for example, $^{18}F$, $^{75}Br$, $^{76}Br$, $^{123}I$, $^{124}I$, $^{125}I$ or $^{134}I$ can be used. When a compound of the formula (1) in which the radioactive halogen substituent, represented by $R^2$ is $^{123}I$, $^{124}I$, $^{123}I$ or $^{131}I$ is produced, $^{123}I$ ion, $^{124}I$ ion, $^{125}I$ ion or $^{131}I$ ion is respectively used as the radioactive halogen ion, and as a compound of the formula (3), preferably used is a compound in which $R^6$ is a non-radioactive iodine, trialkylstannyl substituent having alkyl chains with from 1 to 4 carbon atoms or triphenylstannyl substituent.
When a compound of the formula (1) in which a radioactive halogen substituent represented by $R^2$ is $F^{18}$ is produced, $F^{18}$ ion is used as the radioactive halogen ion, and as a compound of the formulae (3), preferably used is a compound in which $R^6$ is a nitro substituent or trialkylammonium substituent having alkyl chains with from 1 to 4 carbon atoms.

When a compound of the formulae (1) in which a radioactive halogen substituent represented by $R^2$ is $^{75}Br$ or $^{76}Br$ is produced, $^{75}Br$ ion or $^{76}Br$ ion is respectively used as the radioactive halogen ion, and as a compound of the formula (3), preferably used is a compound in which $R^6$ is a non-radioactive bromine.
When a compound of the formula (2) in which a radioactive halogen substituent represented by $R^3$ is $^{1273}I$, $^{124}I$, $^{125}I$ or $^{131}I$ is produced, $^{123}I$ ion, $^{124}I$ ion, $^{125}I$ ion or $^{131}I$ ion is respectively used as the radioactive halogen ion, and as a compound of the formula (4), preferably used is a compound in which $R^7$ is a non-radioactive iodine, trialkylstannyl substituent having alkyl chains with from 1 to 4 carbon atoms or triphenylstannyl substituent.
When a compound of the formula (2) in which a radioactive halogen substituent represented by $R^3$ is $^{18}F$ is produced, $^{18}F$ ion is used as the radioactive halogen ion, and as a compound of the formula (4), preferably used is a compound in which $R^7$ is a nitro substituent or trialkylamonium group having alkyl chains with from 1 to 4 carbon atoms.
When a compound of the formula (2) in which a radioactive halogen substituent represented by $R^3$ is $^{75}Br$ or $^{76}Br$ is produced, $^{75}Br$ ion or $^{76}Br$ ion is respectively used as the radioactive halogen ion, and as a compound of the formula (4), preferable used is a compound in which $R^7$ is non-radioactive bromide.

Also, according to still another aspect of the present invention, a precursor compounds for preparing a radioactive halogen-labeled organic compound, which is represented by the following formulae (3):

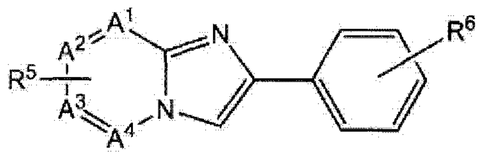

(3)

wherein, $A^1$, $A^2$, $A^3$ and $A^4$ independently represent a carbon or nitrogen,
$R^5$ is a group selected from the group consisting of a hydrogen, hydroxyl group, carboxyl group, sulfuric acid group, amino group, nitro group, cyano group, non-radioactive halogen substituent, alkyl substituent with 1 to 4 carbon atoms and alkoxy substituent with 1 to 4 carbon atoms,
$R^6$ is a group selected from the group consisting of a non-radioactive halogen substituent, nitro group, trialkylstannyl substituent having alkyl chains with 1 to 4 carbon atoms, triphenylstannyl group and trialkylammonium group having alkyl chains with 1 to 4 carbon atoms,
provided that at least one of $A^1$, $A^2$, $A^3$ and $A^4$ represents a carbon, and $R^5$ binds to a carbon represented by $A^1$, $A^2$, $A^3$ or $A^4$,

or a salt thereof is provided.

In the formula (3), the preferable embodiment of $A^1$, $A^2$, $A^3$ and $A^4$ as well as $R^5$ and $R^6$ is the same as those described above concerning the production method.

Also, according to still another aspect of the present invention, a precursor compound for preparing a radioactive halogen-labeled organic compound, which is represented by the following formula (4):

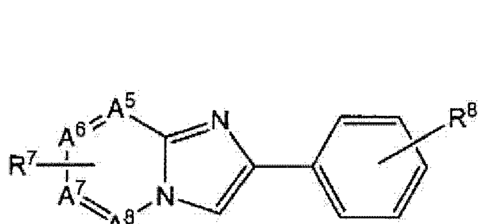

(4)

wherein, $A^5$, $A^6$, $A^7$ and $A^8$ independently represent a carbon or nitrogen,
$R^7$ is a group selected from the group consisting of a non-radioactive halogen substituent, nitro group, trialkylstannyl substituent having alkyl chains with 1 to 4 carbon atoms, triphenylstannyl group and trialkylammonium group having alkyl chains with 1 to 4 carbon atoms, and

is a group selected from the group consisting of a hydrogen, carboxyl group, sulfuric acid group, nitro group, cyano group, non-radioactive halogen substituent, alkyl substituent with 1 to 4 carbon atoms and methoxy substituent, provided that at least one of $A^5$, $A^6$, $A^7$ and $A^8$ represents a carbon, and $R^7$ binds to a carbon represented by $A^5$, $A^6$, $A^7$or $A^8$,

or a salt thereof is provided.

In the formula (4), the preferable embodiment of $A^5$, $A^6$ $A^7$ and $A^8$ as well as $R^7$ and $R^8$ is the same as those described above concerning the production method.

EFFECT OF THE INVENTION

In accordance with the present invention, a reagent for detecting amyloid, which has affinity with amyloid and thus can be used for *in vitro* and *in vivo* detection of amyloid related to a wide range of amyloidosis, can be obtained as well as a production method thereof and a production intermediate thereof.

BESET MODE FOR CARRYING OUT THE INVENTION

(Method for synthesis of a precursor compound for preparing a radioactive halogen-labeled organic compound)

Hereinafter, a method for synthesis of a precursor compound for preparing a radioactive halogen-labeled organic compound according to an aspect of the present invention will be described, taking the case of 2-(4 - tributylstannylphenyl)-6-methoxyimidazo[1,2-a]pyridine.

First, 2-bromo-3-hydroxypyridine is allowed to react with methyl iodide in a presence of sodium methoxide to prepare 2-bromo-3-methoxypyridine. Next, nitration by a mixed acid of conc, sulfuric acid and cone. nitric acid was performed to convert into 2-bromo-3-methoxy-6-nitropyridine, and then reductive elimination of bromo group and reduction of nitro group were performed with palladium carbon to synthesize 2-amino-5-methoxypyridine (Fig. 1, Steps 1-3). In this series of reaction, the reaction can be conducted in accordance with ordinary methods, for example, the method described in a literature, Joseph G. Lombardino, Journal of Medical Chemistry, 1981, 24, p.39-42.

The resulting 2-amino-5-methoxypyridine is allowed to react with 2-bromo-4 -bromoacetophenone to brominate 4 -position to prepare 2-(4 -bromophenyl)-6-methoxyimidazo[1,2-a]pyridine (Fig. 1, Steep 4). This step can be done according to the following procedure.

First, 2-promo-4 -bromoacetophenone and 2-amino-5-methoxypyridine are dissolved in an inactive solvent such as acetonitrile, and are allowed to react with each other at a reflux temperature for 2 to 6 hours to produce 2-(4 -bromophenyl)-6-methoxyimidazo[1,2-a]pyridine hydrobromide salt as white precipitates. The solvent used in this instance may be acetonitrile or another solvent that is usually employed in a similar reaction, for example, methanol and acetone. The reaction, temperature may be a temperature allowing refluxing, for example, 90°C when the solvent is acetonitrile. The amount of the solvent to be used may be an amount sufficient to effect the reaction, however, it should be noted that if the solvent is too much, it will become difficult to obtain precipitates of reaction products. For example, when 2-promo-4 -bromoacetophenone in an amount corresponding to 10 mmol is used for the reaction, the amount of a solvent to be used can be about 40 to 50 mL.

Next, the reaction solution is filtered to recover the precipitates. The while precipitates are suspended in a mixed solution of methanol/water (1:1). Then, an aqueous saturated solution of sodium hydrogencarbonate is added thereto in a very excessive amount relative to the suspended precipitates to release 2-(4 -bromophenyl)-6-methoxyimidazo[1,2-a]pyridine as precipitates. The newly generated precipitates are filtered to recover 2-(4 - bromophenyl)-6-methoxyimidazo[1,2-a]pyridine as the target compound in this step. The amount of the mixed solution of water/methanol is not specifically limited as long as it is sufficient to effect the reaction. However, it should be noted that if the amount of the mixed solution is too much, precipitation of crystals will be hindered. For example, when 2-bromo-4 -bromoacetophenone in an amount corresponding to 10 mmol is used, the mixed solution of water/methanol may be used in an amount of about 40 to 100 mL. The amount of sodium hydrogencarbonate is not specifically limited as long as it is very excessive relative to the above-described precipitates as reaction substrates. For example, when the reaction is affected under the above-described conditions, the amount of an aqueous saturated solution of sodium hydrogencarbonate to be added to the reaction solution can be about 25 mL.

The resulting 2-(4 -bromophenyl)-6-methoxyimidazo[1,2-a]pyridine was dissolved in dioxane, and after triethylamine is added, bis(tributyltin) and a catalytic amount of tetrakis-triphenylphosphine palladium are added. This reaction mixture is heated at about 90°C and reacted, and then a solvent is distilled off and a chromatographic purification is performed to obtain 2-(4 -tributylstannylphenyl)-6-methoxyimidazo[1,2-a]pyridine as the target compound (Fig. 1, Step 5). The amount of bis(tributyltin) to be used is an amount satisfying a condition where it is excessive relative to the reaction substrate, specifically, it is preferably about 1.5 times in molar ratio relative to the 2-(4 - bromophenyl)-6-methoxyimidazo[1,2-a]pyridine as the reaction substrate.

When a compound with a substituent at the 4 - position being a trialkylstannyl substituent other than tributylstannyl substituent is obtained, various bis(trialkyltin)s that fit purposes can be used instead of bis(tributyitin) in Step 5. For example, when a compound having a trimethylstannyl substituent as a substituent at the 4 -position is synthesized, a reaction similar to the above can be performed in Step 4 using bis(trimethyltin).

In addition, a compound with a substituent attached to imidazopyridine ring being a hydroxyl substituent can be obtained by, for example, allowing 2-(4 -bromophenyl)-6-methoxyimidazo[1,2-a]pyridine obtained in the Step 4 to react with tribromoboron to conduct demethylation, and then conducting a reaction of the Steep 5. Also, when a compound with a substituent attached to imidazopyridine ring being a methyl substituent and ethoxy substituent, 2-amino-5-methylpyridine and 2-amino-5-ethoxypyridine can be respectively used instead of 2-'amino-5-methoxypyridine used in the Step 4.

A compound with an imidazopyridine ring in which the binding site for the functional group is a carbon atom other than the carbon at 6-position can be obtained by using a compound with a pyridine ring to which alkoxy substituent and the like is bonded at a different site instead of 2-amino-5-methoxypyridine used in Step 4. For example, when a binding site for the functional group is the carbon at 8-position in the imidazopyridine ring in the above Example, 2-amino-3-methoxypyridine may be used instead of 2-amino-5-methoxypyridine in Step 4.

Further, a labeling precursor compound with a radioactive halogen-labeled site being imidazopyridine ring, for example, 6-tributylstannyl-2-(4 - methoxyphenyl)imidazo[1,2-a]pyridine can be prepared according to the following procedure.

First, 4 -hydroxyacetophenone is allowed to react with cupric bromide in accordance with ordinary methods, for example, the method described in a literature, King, L. Carroll and Ostrum, G. Kenneth, Journal of Organic Chemistry, 1964, 29(12), p.3459-3461, to prepare 2-bromo-4 -hydroxyacetophenone (Fig. 3, Step 1). This is allowed to react with 2-amino-5-iodopyridine in an inert solvent such as acetonitrile, and the resulting precipitates are purified to obtain 2-(4 hydroxyphenyl)-6-iodoimidazo[1,2-a]pyridine (Fig. 3, Step 2). A condition in this step may be used in the same manner as above in Fig. 1, Step 4.

Next, the resulting 2-(4 hydroxyphenyl)-6-iodoimidazo[1,2-a]pyridine is dissolved in a mixed solution of methanol and dioxane, trimethylsilyldiazomethane is added thereto to effect reaction, and s solvent is distilled to purify the residue to prepare 6-indo-2-(4 - methoxyphenyl)imidazo[1,2-a]pyridine (Fig. 3, Step 3). In this step, the amount of trimethylsilyldiazomethane is an amount equivalent to or more of 2-(4 hydroxyphenyl-6-iodoimidazo[1,2-a]pyridine.

The resulting 6-iodo-2-(4 - methoxyphenyl)imidazo[1,2-a]pyridine was dissolved in dioxane, and after triethylamine is added, bis(tributyltin) and a catalytic amount of tetrakis-triphenylphosphine palladium are added to effect the reaction to obtain 6-tributylstannyl-2-(4 - methoxyphenyl)imidazo[1,2-a]pyridine as the target compound (Fig. 4, Step 1). The amount of bis(tributyltin) to be used is an amount satisfying a condition where it is excessive relative to the reaction substrate, specifically, it is preferably about 1.5 times in molar ratio relative to the 2-(4 -bromophenyl)-6-methoxyimidazo[1,2-a]pyridine as the reaction substrate. A condition in this step may be used in the same manner as described above in Fig. 1, Step 5.

(A method for synthesis of a radioactive halogen-labeled organic compound)

Hereinafter, a method for production of a radioactive halogen-labeled organic compound according to another aspect of the present invention will be described, taking the case of radioactive iodine-labeled compounds.

The synthesis of radioactive iodine-labeled compounds can be performed by dissolving the labeling precursor compound prepared as above procedure in an inert organic solvent, adding thereto a [$^{123}$I]sodium iodide solution obtained by known methods as a radioactive halogen ion, adding an acid and an oxidizing agent thereto to effect reaction. As an inert organic solvent dissolving the labeling precursor compound, various solvents having no reactivity with the labeling precursor, [$^{123}$I]sodium iodide and the like can be used, and preferably ethanol can be used.

As the acid, may be used various ones, and preferably hydrochloric acid.
The oxidizing agent is not particularly limited as long as it can effect the oxidation of iodine in the reaction solution, and is preferably hydrogen peroxide or peracetic acid. The amount of the oxidizing agent to be added may be an amount sufficient to oxidize iodine in the reaction solution.

A compound labeled with a radioactive halogen other than iodine can be synthesized by labeling a labeling precursor that fits a purpose of synthesis with a radioactive halogen that fits the purpose. For example, in order to synthesize 2-(4 - [$^{18}$F]fluorophenyl)imidazo[1,2-a]pyridine, the labeling precursor 2-(4 -nitrophenyl)-6-methoxyimidazo [1,2-a]pyridine can be reacted with [$^{18}$F]fluoride ion in the presence of a phase transfer catalyst and potassium carbonate.

(Methods for preparing and using a detection reagent in accordance with the present invention)

Amyloid has manly different structures depending upon kinds of precursor protein, but they are common in a

point of having β-sheet structure. It has been known that many staining reagents for amyloid such as Thioflavin T and Congo-red target at the β-heet structure, and has the staining ability equally to different kinds of amyloid.
The compounds of the formulae (1) and (2) according to the present invention have affinity with amyloid originated from amyloid β-rotein (hereinafter referred to as Aβ) as a precursor compound, and also has an activity of inhibiting the binding to amyloid of Thioflavin T which is known to bind to a wide range of amyloid.
Therefore, the compounds of the formulae (1) and (2) according to the present invention are considered to have affinity with β-sheet structure of amyloid protein like thioflavin T. This suggests that the compounds of the formulae (1) and (2) according to the present invention have the same affinity with various amyloids.
That is, the reagent for detecting amyloid according to the present invention can be used for diagnosing a the systemic amyloidosis and localized amyloidosis similarly to Thioflavin T and Congo-red. The syatematic amyloidosis includes immunoglobulin amyloidosis, reactive AA amyloidosis, familial amyloidosis, dialysis amyloidosis and senile amyloidosis can be included. The localized amyloidosis includes brain amyloidosis, endocrine amyloidosis, cutaenous amyloidosis and localized nodular amyloidosis.

The reagent for detecting amyloid according to the present invention can be not only used as a reagent used in vitro for biopsy, but also used as a reagent used *in vivo* as a radioactive diagnostic agent.

The reagent for detecting amyloid according to the present invention can be prepared as a solution which comprises the radioactive halogen-labeled compound of the above formula (1) or (2) blended in water, a physiological saline solution or a Ringer's solution optionally adjusted to an appropriate pH, like other commonly-known radioactive diagnostic agents. In this instance, concentration of the present compound should be adjusted to not more than the concentration at which stability of the present compound is ensured. Dosage of the present compound is not specifically limited as long as it is sufficient to obtain an image of distribution of an administered agent. For example, in case of iodine-123-labeled compound and fluorine-18-labeled compounds, about 50 to 600 MBq per adult body of 60 kg weight can be administered intravenously or loyally. Distribution of administered agents can be imaged by known methods. For example, iodine-123-labeled compounds can be imaged by a SPECT apparatus while fluorine-18-labeled compounds can be imaged by a PET apparatus.

By administering the reagent for detecting amyloid according to the present invention to a living body, amyloid which is deposited in biological tissues such as brain, heart, lung, digestive tract, blood vessel, liver, pancreas, kidney, joint and bones can be imaged, and it is useful for imaging amyloid deposition in biological tissues difficult in biopsy collection, for example, brain heat, lung, pancreas, bones and joints.

## EXAMPLE

Hereinafter, the present invention is described below in more detail by way of Examples, Comparative Examples and Reference Examples. However, these Example never limit the scope of the present invention.

(Example 1) Synthesis of 2-(4 -tributylstannylphenyl)-6-methoxyimidazo[12-a]pyridine

100.0 g (corresponding to 0.575 sol) of 2-bromo-3-hydroxypyridine was dissolved in 310 mL of dimethylsulfoxide, and 575 mL (corresponding to 0.575 mol) of 1 mol/L sodium methoxide-methanol solution was added thereto. Then, the reaction solution was heated to 90°C to distill off methanol. After the reaction solution was cooled down to 10°C or lower, 93.9 g (corresponding to 0.662 mol) of methyl iodide was added, and then stirred at room temperature for 20.5 hours. After the completion of the reaction, the reaction solution was poured into ice water and extracted twice with chloroform. The combined chloroform layer was washed with 1 mol/L sodium hydroxide solution, washed twice with a saturated sodium chloride solution. The resultant was dried over anhydrous sodium sulfate, and then the solvent was distilled off under reduced pressure, to obtain 65.4 g (corresponding to 0.348 mol) of 2-bromo-3-methoxypyridien (Fig. 1, Step 1).

262 mL of conc. sulfuric acid was cooled down to - 2°C, and 262 mL of 90 % nitric acid was carefully added thereto. Subsequently, 65.3 g (corresponding to 0.347 mmol) of 2-bromo-3-methoxypyridine was carefully added thereto. After the resulting mixture was stirred in an ice bath for 10 minutes, the mixture was stirred at room temperature for 30 minutes, and then was heated to 55°C and further stirred for 1.5 hours. After the reaction solution was cooled, the reaction solution was poured little by little into crushed ice to generate precipitates. The precipitates were filtered and washed with water, and then dried over phosphorous pentoxide under reduced pressure, to obtain 55.7 g (corresponding to 0.239 mmol) of 2-bromo-3-methoxy-6-nitropyridine (Fig. 1, Step 2).

55.6 g (corresponding to 0.239 mol) of 2-bromo-3-methoxy-6-nitropyridine was dissolved in 1700 mL of ethanol, and 37.3 g (50% wet) of 10 % palladium-carbon was added thereto under argon stream. To the mixture, 283 mL of hydrazine monohydrate was added dropwise. After the reaction mixture was refluxed for 70 minutes, the reaction solution was cooled to room temperature. Then, after palladium-carbon was filtered off, the residue was washed with ethanol, and the washing were combined with the filtrate. The combined solution was concentrated under reduced pressure. The, 1300 mL of water and 130 mL of conc. aqueous ammonia were added to the concentrate, and the resulting mixture was extracted eight times with chloroform. The combined chloroform layer was dried over anhydrous sodium sulfate and concentrated under reduced pressure. The resulting crude product was distilled under reduced pressure to obtain

26.2 g (corresponding to 0.211 mol) of 2-amino-5-methoxypyridine (Fig. 1, Step 3).

844 mg (corresponding to 3.0 mmol) of 2-bromo-4 - bromoacetophenone and 378 mg (corresponding to 3.0 mmol) of 2-amino-5-methoxypyridine were dissolved in 25 mL of acetonitrile. The resulting solution was refluxed in an oil bath at 105°C for 3.5 hours. After the completion of the reaction, the reaction solution was cooled down to room temperature, and precipitates were filtered and recovered. The precipitates were washed with acetonitrile and dried under reduced pressure to obtain crude crystals. The resulting crude crystals were suspended in a mixed solution of 7 mL of water and 7 mL of methanol. Then, about 7 mL of a saturated sodium hydrogencarbonate solution was added thereto, and the mixture was sonicated for 5 minutes using an ultrasonic washing machine. Precipitates were filtered and recovered, sufficiently washed with water, and dried under reduced pressure, to obtain 640 mg (corresponding to 2.11 mmol) of 2-(4 -bromophenyl)-6-methoxyimidazo[1,2-a]pyridine (Fig. 1, Step 4).

463 mg (corresponding to 1.5 mmol) of 2-(4 - bromophenyl)-6-methoxyimidazo[1,2-a]pyridine was dissolved in 25 mL of dioxane, and 2 mL of triethylamine was added thereto. Then, 1.15 mL (corresponding to 2.25 mmol) of bis (tributyltin) and 19 mg (at a catalytic amount) of tetrakis-triphenylphosphine palladium were added. After the reaction mixture was stirred at 90°C for 15 hours, the solvent was distilled off under reduced pressure. The residue was purified by preparative TLC (elution solvent: hexane/ethyl acetate = 5/1 to 4/1). Further, the resulting crude product was purified by recycle preparative HPLC (HPLC apparatus: LC-908 (under trade name; manufactured by Nippon Bunseki Kogyo); column: two of JAIGEL 2H (under trade name; manufactured by Nippon Bunseki Kogyo) connected together; mobile phase: chloroform), to obtain 419 mg (corresponding to 0.82 mmol) of 2-(4 -tributylstannylphenyl)-6-methoxyimidazo [1-2-a]pyridine (Fig. 1, Step 5).

The NMR measurement results of the resulting 2-(4 - tributylstannylphenyl)-6-methoxyimidazo[1,2-a]pyridine (internal standard: tetramethylsilane) are shown below.

NMR apparatus employed: JNM-ECP-500 (manufactured by Japan Electron Optics Laboratory Co., Ltd. (JEOL))

$^1$H-NMR (solvent: chloroform-d1, resonance frequency: 500 MHz): δ 7.89-7.84 (m, 2H), 7.81 (s, 1H), 7.66-7.63 (m, 1H), 7.57-7.46 (m, 3H), 6.96 (dd, J = 9.7, 2.4 Hz, 1H), 3.83 (s, 3H), 1.64-1.47 (m, 6H), 1.34 (sextet, J = 7.3 Hz, 6H), 1.15-1.00 (m, 6H), 0.89 (t, J = 7.3 Hz, 9H).

$^{13}$C-NMR (solvent: chloroform-dl, resonance frequency: 125 MHz): δ 149.29, 146.02, 142.90, 136.84, 133.52, 125.23, 119.66, 117.73, 109.16, 107.47, 56.20, 29.12, 27.38, 13.69, 9.62.

Example 2) Synthesis of [$^{123}$I]-2-(4 -iodophenyl)-6-methoxyimidazo[1,2-a]pyridine]

To 75 μL of a solution of 2-(4 - tributylstannylphenyl)-6-methoxyimidazo[1,2-a]pyridine in methanol (concentration: 1 mg/mL), 100 μL of 1 mol/L hydrochloric acid, 10 μL of 1 mmol/L sodium iodide, [$^{123}$I]sodium iodide solution of 160 MBq (80 μL in volume) and 20 μL of 10% (w/v) hydrogen peroxide were added. After the mixed solution was left to stand at 50°C for 10 minutes, the solution, was subjected to HPLC under the following conditions, to obtain [$^{123}$I]-2-(4 -iodophenyl)-6-methoxyimidazo[1,2-a]pyridine fraction.

HPLC conditions:

column: Phenomenex Luna C18 (trade name; manufactured by Phenomenex Co.; size: 4.6 x 150 mm)
Mobile phase: 0.1 % trifluoroacetic acid in water/0.1 % trifluoroacetic acid in acetonitrile = 80/20 to 0/100 (17 minutes, linear gradient)
Flow rate: 1.0 mL/min.
Detector: Ultraviolet visible absorptiometer (Detection wavelength: 282 nm) and radioactivity counter (manufactured by raytest: type STEFFI)

10 ml of water was added to the faction. The resulting solution was passed through a reversed phase column (trade name: Sep-Pak (registered trademark) Light C18 Cartridges manufactured by Waters: the packed amount of the packing agent: 145 mg) so that the column adsorbs and collects [$^{123}$I]-2-(4 -iodophenyl)-6-methoxyimidazo[1,2-a]pyridine. The column was rinsed with 1 mL of water, and then 1 mL of diethylether was passed therethrough to elute [$^{123}$I]-2-(4 -iodophenyl)-6-methoxyimidazo[1,2-a]pyndine. The amount of radioactivity of the obtained compound was 27 MBq (immediately after the production). Further, the TLC analysis was conducted under the following conditions, and as a result, the radiochemical purity of the compound was 97%.

TLC analysis conditions:

TLC plate: RP-18F254 (trade name; manufactured by Merck & Co., Inc.)
Mobile phase: Chloroform/methanol/triethylamine = 100/1/2
Detector: Bio-imaging Analyzer, BAS-2500 (type: BAS-2500 manufactured by FUJIFILM Corporation)

(Example 3) Synthesis of [$^{125}$I]-2-(4 -iodophenyl)-6-methoxyimidazo[1,2-a]pyridine

To 75 μL of a solution of 2-(4-tributylstannylphenyl)-6-methoxyimidazo[1,2-a]pyridine in methanol (concentration: 1 mg/mL), 100 μL of 1 mol/L hydrochloric acid, 10 μL of 1 mmol/L sodium iodide, [$^{123}$I]sodium iodide solution of 32 MBq (20 μL in volume) and 20 μL of 10% (w/v) hydrogen peroxide were added. After the mixed solution was left to stand at 50°C for 10 minutes, the solution was subjected to HPLC under the following conditions, to obtain [$^{125}$I]-2-(4 -iodophenyl)-6-methoxyimidazo[1,2-a]pyridine fraction.

10 ml of water was added to the fraction. The resulting solution was passed through a reversed phase column (trade name: Sep-Pak (registered trademark) Light C18 Cartridges manufactured by Waters: the packed amount of the packing agent: 145 mg) so that the column adsorbs and collects [$^{125}$I] -2-(4 -iodophenyl)-6-methoxyimidazc[1,2-a]pyridine. The column was rinsed with 1 mL of water, and then 1 mL of diethylether was passed therethrough to elute [$^{125}$I]-2-(4 -iodophenyl)-6-methoxyimidazo[1,2-a]pyridine. The amount of radioactivity of the obtained compound was 15 MBq (immediately after the production). Further, the TLC analysis was conducted under the following conditions, and as a result, the radiochemical purity of the compound was 92%.

(Example 4) Synthesis of 2-(4 -iodophenyl)-6-methoxyimidazo[1,2-a]pyridine (non-iodinated form)

100 mg (corresponding to 0.20 mmol) of 2-(4-tributylstannylphenyl)-6-methoxyimidazo[1,2-a]pyridine obtained from Example 1 was dissolved in 2.0 mL of dichloromethane, to which 37 mg (corresponding to 0.29 mmol) of iodine was added. The reaction mixture was stirred at the temperature of 0°C for 30 minutes, and then, a saturated aqueous sodium hydrogencarbonate solution and a saturated aqueous sodium thiosulfate solution were added thereto, and extracted with dichloromethane for three times. The combined dichloromethane layer was dried, and then concentrated under reduced pressure. The resulting crude product was purified by silica gel column chromatography (elution solvent: hexane/ethyl acetate = 2/1), to obtain 44.5 mg (corresponding to 0.13 mmol) of 2-(4 -iodophenyl)-6-methoxyimidazo [1,2-a]pyridine (Fig. 2, Step 1).

The NMR measurement results of the resulting 2-(4 - iodophenyl)-6-methoxyimidazo[1,2-a]pyridine (internal standard: tetramethylsilane) are shown below.

NMR apparatus employed: JNM-ECP-500 (manufactured by Japan Electron Optics Laboratory Co., Ltd. (JEOL)) $^{1}$H-NMR (solvent: chloroform-d1, resonance frequency: 500 MHz): δ 7.75-7.71 (m, 3H), 7.64-7.62 (m, 2H), 7.59 (d, J = 1.8 Hz, 1H), 7.49 (d, J = 10.1 Hz, 1H), 6.96 (dd, J = 10.1, 1.8 HZ, 1H), 3.81 (s, 3H).

$^{13}$C-NMR (solvent: chloroform-d1, resonance frequency: 125 MHz): δ 149.80, 144.94, 143.27, 138.12, 133.91, 127.88, 120.56, 118.05, 109.72, 107.79, 93.45, 56.57.

(Example 5) Synthesis of 6-iodo-2-(4 - methoxyphenyl)imidazo[1,2-a]pyridine (non-iodinated form)

50 mL of ethyl acetate was added to 28.17 g (corresponding to 126 mmol) of cupric bromide to obtain a suspension, to which a solution of 8.18 g (corresponding to 60.0 mmol) of 4 -hydroxyacetophenone in a mixed solution of 50 mL of ethyl acetate and 50 mL of chloroform was added. Then, the resulting mixture was refluxed. After 5 hours, the reaction solution was cooled down to room temperature and filtered. The resulting filtrate was concentrated under reduced pressure. The residue was dissolved in ethyl acetate and subjected to decoloring operation with addiction of active charcoal. Then, the resulting solution was filtered and concentrated. The resulting crude product was purified by flash silica gel column chromatography (elution solvent: chloroform/methanol = 20/1), and recrystallized from methyl acetate/petroleum ether, to obtain 7.25 g (corresponding to 33.7 mmol) of 2-bromo-4 - hydroxyacetophenone (Fig. 3, Step 1).

441 mg (corresponding to 2.0 mmol) of 2-bromo-4 - hydroxyacetophenone and 449 mg (corresponding to 2.0 mmol) of 2-amino-5-iodopyridine were dissolved in 15 mL of acetonitrile. The resulting solution was refluxed in an oil bath at 110°C for 5 hours. After the complexion of the reaction, the reaction solution was cooled down to room temperature, and precipitates where filtered and recovered. The precipitates were washed with acetonitrile and dried under reduced pressure. The resulting crude crystals were suspended in a mixed solution of 10 mL of water and 10 mL of methanol. Then, about 10 mL of a saturated sodium hydrogencarbonate solution was added thereto, and the mixture was sonicated for 5 minutes using an ultrasonic washing machine. Precipitates were filtered and recovered from the resulting mixture, sufficiently washed with water, and dried under reduced pressure, to obtain 0.53 g (corresponding to 1.56 mmol) of 2-(4-hydroxyphenyl)-6-iodoimidazo[1,2-a]pyridine (Fig. 3, Step 2).

0.40 g (corresponding to 1.2 mmol) of 2-(4 - hydroxyphenyl)-6-iodoimidazo[1,2-a]pyridine was dissolved in a mixed solution of 30 mL of ethanol and 50 mL of dioxane, and 1.2 mL (corresponding to 2.4 mmol) of trimethylsilyldiazomethane (2M hexane solution) was added thereto. The reaction mixture was stirred at room temperature for 24.5 hours, and then a solvent was distilled under reduced pressure. The residue was recrystallized from ethyl acetate to obtain 223 mg (corresponding to 0.64 mmol) of 6-iodo-2-(4-methoxyphenyl)imidazo[1,2-a]pyridine.

The NMR measurement results of the resulting 6-iodo-2-(4 -methoxyphenyl)imidazo[1,2-a]pyridine (internal standard: tetramethylsilane) are shown below.

NMR apparatus employed: JNM-ECP-500 (manufactured by Japan Electron Optics Laboratory Co., Lid. (JEOL)) $^{1}$H-NMR (solvent: chloroform-d1, resonance frequency: 500 MHz): δ 8.37-8.34 (m, 1H), 7.88-7.84 (m, 2H), 7.72 (s, 1H), 7.40 (d, J = 9.4 Hz, 1H), 7.31 (dd, J = 9.4, 1.6 HZ, 1H), 6.99-6.95 (m, 2H), 3.86 (s, 3H).

$^{13}$C-NMR (solvent: chloroform-dl, resonance frequency: 125 MHz): δ 159.86, 146.32, 144.18, 132.32, 130.28, 127.41, 125.90, 118.32, 114.22, 106.88, 74.76, 55.33.

(Examples 6) Synthesis of 6-tributylstannyl-2-(4 - methoxyphenyl)imidazo[1,2-a]pyridine

87.6 mg (corresponding to 0.25 mmol) of 6-iodo-2-(4 -methoxyphenyl)imidazo[1,2-a]pyridine obtained from Example 5 was dissolved in 10 mL of dioxane, and 2 mL of triethylamine was added thereto. Then, 190 μL (corresponding to 0.375 mmol) of bis(tributyltin) and 20 mg (at a catalytic amount) of tetrakis-triphenylphosphine palladium were added. After the reaction mixture was stirred at 90°C for 21.5 hours, the solvent was distilled off under reduced pressure. The residue was purified by flash silica gel column chromatography (elution solvent: hexane/ethyl acetate = 3/1). Further, the resulting crude product was purified by recycle preparative HPLC (HPLC apparatus: LC-908 (under trade name; manufactured by Nippon Bunseki Kogyo); column: two of JAIGEL 2H (under trade name; manufactured by Nippon Bunseki Kogyo) connected together; mobile phase: chloroform), to obtain 53 mg (corresponding to 0.10 mmol) of 6-tributylstannyl-2-(4-methoxyphenyl)imidazo[1,2-a]pyridine (Fig. 4, Step 1).

The NMR measurement results of the resulting 6-tributylstannyl-2-(4 -methoxyphenyl)imidazo[1,2-a]pyridine (internal standard: tetramethylsilane) are shown below.

NMR apparatus employed: JNM-ECP-500 (manufactured by Japan Electron Optics Laboratory Co., Ltd. (JEOL)) $^1$H-NMR (solvent: chloroform-dl, resonance frequency: 500 MHz): δ 8.01-7.93 (m, 1H), 7.92-7.87 (m, 2H), 7.74 (s, 1H), 7.60-7.56 (m, 1H), 7.18-7.09 (m, 1H), 6.99-6.94 (m, 2H), 3.84 (s, 3H), 1.66-1.46 (m, 6H), 1.35 (sextet, J = 7.3 Hz, 6H), 1.19-1.02 (m, 6H), 0.91 (t, J = 7.3 Hz, 9H).

$^{13}$C-NMR (solvent: chloroform-d1, resonance frequency: 125 MHz): δ 159.45, 145.56, 145.01, 131.00, 129.95, 127.22, 126.70, 121.69, 116.80, 114.06, 106.24, 55.23, 27.24, 13.59, 9.74.

(Example 7) Synthesis of [$^{123}$I]-6-iodo-2-(4 - methoxyphenyl)imidazo[1,2-a]pyridine

To 50 μL of a solution of 6-tributylstannyl-2-(4 - methoxyphenyl)imidazo[1,2-a]pyridine in ethanol (concentration: 1 mg/mL), 50 μL of 1 mol/L hydrochloric acid, 10 μL of 1 mmol/L sodium iodide, [$^{123}$I]sodium iodide, solution of 318 MBq (50 μL in volume) and 10 μL of 10% (w/v) hydrogen peroxide were added. After the mixed solution was left to stand at 50°C for 10 minutes, the solution was subjected to HPLC under the same conditions as described in Example 2, to obtain [$^{123}$I]-6-iodo-2-(4 methoxyphenyl)imidazo[1,2-a]pyridine fraction.

10 ml of water was added to the fraction. The resulting solution was passed through a reversed phase column (trade name: Sep-Pak (registered trademark) Light C18 Cartridges manufactured by Waters: the packed amount of the packing agent: 130 mg) so that the column adsorbs and collects [$^{123}$I]-6-iodo-2-(4-methoxyphenyl)imidazo[1,2-a]pyridine. The column was rinsed with 1 mL of water, and then 1 mL of diethylether was passed therethrough to elute [$^{123}$I]-6-iodo-2-(4 - methoxyphenyl)imidazo[1,2-a]pyridine. The amount of radioactivity of the obtained compound was 86 MBq (immediately after the production). Further, the TLC analysis was conducted under the same conditions as described in Example 2, and as a result, the radiochemical purity of the compound was 98%.

(Example 8) Synthesis of 2-(4-fluorophenyl)-6-iodoimidazo[1,2-a]pyridine (non-iodinated form)

40 mL of ethyl acetate was added to 3.70 g (corresponding to 16.6 mmol) of cupric bromide to obtain suspension, and 1.0 mL (corresponding to 8.27mmol) of 4-fluoroacetophenone was added thereto. Then, the resulting mixture was refluxed. After 3 hours, the reaction mixture was cooled down to room temperature and filtered. The resulting filtrate was concentrated under reduced pressure. The residue was dissolved in ethyl acetate and concentrated. The resulting crude product was purified by silica gel column chromatography (elution solvent: hexane/ethyl acetate = 10/1), to obtain 1.82 g (corresponding to 8.39 mmol) of 2-bromo-4 - fluoroacetophenone (Fig. 5, Step 1).

1.82 g (corresponding to 8.39 mmol) of 2-bromc-4 - fluoroacetophenone and 1.29 g (corresponding to 5.87 mmol) of 2-amino-5-iodopyridine were dissolved in 40 mL of acetonitrile. The resulting solution was refluxed in an oil bath at 110°C for 1.5 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 20 mL of water and 10 mL of methanol. Then, about 30 mL of a saturated sodium hydrogencarbonate solution was added thereto, and the mixture was sonicated for 5 minutes using an ultrasonic washing machine. Precipitates were filtered and recovered from the resulting mixture, sufficiently washed with water, and dried under reduced pressure, to obtain 1.28 g (corresponding to 3.79 mmol) of 2-(4-fluorophenyl)-6-iodoimidazo[1,2-a]pyridine (Fig. 5, Step 2).

The NMR measurement results of the resulting 2-(4-fluorophenyl)-6-iodoimidazo[1,2-a]pyridine (internal standard: tetramethylsilane) are shown below.

NMR apparatus employed: JNM-ECP-500 (manufactured by Japan Electron Optics Laboratory Co., Ltd. (JEOL)) $^1$H-NMR (solvent: dimethylformamide-d7, resonance frequency: 500 MHz): δ 9.06 (s, 1H), 8.44 (s, 1H), 8.01-7.98 (m, 2H), 7.72 (d, J = 9.6 Hz, 1H), 7.57 (d, J = 9.6 Hz, 1H), 7.38-7.34 (m, 2H).

(Example 9) Synthesis of 6-tributylstannyl-2-(4 - fluorophenyl)imidazo[1,2-a]pyridine

338 mg (corresponding to 1.00 mmol) of 2-(4-fluorophenyl)-6-iodoimidazo[1,2-a]pyridine was dissolved in 4.0 mL of dioxane, and 2 mL of triethylamine was added thereto. Then, 0.76 mL (corresponding to 1.5 mmol) of bis(tributyltin) and 76.3 mg (at a catalytic amount) of tetrakis-triphenylphosphine palladium were added. After the reaction mixture was stirred at 90°C for 18 hours, the solvent was distilled off under reduced pressure. The residue was purified by flash silica gel column chromatography (elution solvent: hexane/ethyl acetate = 5/1), to obtain 310 mg (corresponding to 0.618

mmol) of 6-tributylstannyl-2-(4 -fluorophenyl)imidazo[1,2-a]pyridine (Fig. 6, Step 1).

The NMR measurement results of the resulting 6-tributylstannyl-2-(4 -fluorophenyl)imidazo[1,2-a]pyridine (internal standard: tetramethylsilane) are shown below.

NMR apparatus employed: JNM-ECP-500 (manufactured by Japan Electron Optics Laboratory Co., Ltd. (JEOL)) $^1$H-NMR (solvent: chloroform-d1, resonance frequency: 500 MHz): δ 7.98 (s, 1H), 7.94-7.90 (m, 2H), 7.77 (s, 1H), 7.60-7.58 (m, 1H), 7.17-7.10 (m, 3H), 1.64-1.48 (m, 6H), 1.35 (sextet, J = 7.3 Hz, 6H), 1.19-1.05 (m, 6H), 0.91 (t, J = 7.3 Hz, 9H).

$^{13}$C-NMR (solvent: chloroform-d1, resonance frequency: 125 MHz): δ 162.7 (d, $^1J_{CF}$ = 246.7 Hz), 145.7, 144.3, 131.4, 130.3 (d, $^4J_{CF}$ = 2.9 Hz), 130.1, 127.7 (d, $^3J_{CF}$ = 8.6 Hz), 122.2, 117.1, 115.6 (d, $^2J_{CF}$ = 21.1 Hz), 106.9, 29.0, 27.3, 13.7, 9.8.

(Example 10) Synthesis of [$^{123}$I]-2-(4 -fluoropheny)-6-iodoimidazo[1,2-a]pyridine

To 60 μL of a solution of 6-tributylstannyl-2-(4 - fluorophenyl)imidazo[1,2-a]pyridine (concentration: 1 mg/mL) in a mixed solution of methanol/dimethylsulfoxide (mixing ratio = 9/1), 40 μL of 1 mol/L hydrochloric acid, 15 μL of 1 mmol/L sodium iodide, 12 μL of [$^{123}$I]sodium iodide of 150.1 MBq and 15 μL of 10% (w/v) hydrogen peroxide were added. After the mixed solution was left to stand at 50°C for 10 minutes, the solution was subjected to HPLC under the following conditions, to obtain 2-(4-fluorophenyl)-6-[$^{123}$I]iodoimidazo[1,2-a]pyridine fraction.

HPLC conditions:

column: Phenomenex Luna C18 (trade name; manufactured by Phenomenex Co.; size: 4.6 x 150 mm)
Mobile phase: 0.1 % trifluoroacetic acid/0.1 % acetonitrile = 20/80 to 0/100 (17 minutes)
Flow rate: 1.0 mL/min.
Detector: Ultraviolet visible absorptiometer (Detection wavelength: 282 nm) and radioactivity counter (manufactured by raptest: type STEFFI)

10 ml of water was added to the fraction. The resulting solution was passed through Sep-Pak coloumn (trade name: Sep-Pak (registered trademark) Light C18 Cartridges manufactured by Wasters: the packed amount of the packing agent: 145 mg) so that the column adsorbs and collects [$^{123}$I-2-(4 -fluorophenyl)-6-iodoimidazo[1,2-a]pyridine. The column was rinsed with 1 mL of water, and then 1 mL of diethylether was passed therethrough to elute 2-(4 -fluorophenyl)-6-[$^{123}$I]iodoimidazo[1,2-a]pyridine. The amount of radioactivity of the obtained compound was 28.8 MBq (immediately after the production).

(Reference Example 1) Synthesis of [$^{123}$I]-IMPY

[$^{123}$I]-IMPY was prepared in accordance with the following steps for measurement of $logP_{octanol}$ and evaluations on accumulations in brain.

In accordance with the literature (Zhi-Ping Zhuang et al., J. Med. Chem, 2003, 46, p.237-243), 6-tributylstannyl-2-[4 -(N,N-dimethylamino)phenyl]4-midazo[1,2-a]pyridine was synthesized, and dissolved in methanol (concentration: 1mg/mL). To 53 μL of the resulting solution, 75 μL of 1 mol/L hydrochloric acid, 60-70 μL of [$^{123}$I]sodium iodide of 224-253 MBq, 10 μL of a 1 mmol/L sodium iodide solution and 15 μL of 10% (w/v) hydrogen peroxide were added. After the mixed solution was left to stand at 50°C for 10 minutes, the solution was subjected to HPLC under the same conditions as described in Example 2, to obtain [$^{123}$I]-IMPY fraction.

10 ml of water was added to the fraction. The resulting solution was passed through a reversed phase column (trade name: Sep-Pak (registered trademark) Light C18 Cartridges manufactured by Waters; the packed amount of the packing agent: 130 mg), so that the column adsorbs and collects the [$^{123}$I]-IMPY. The column was rinsed with 1 mL, of water, and then 1 mL of diethylether was passed therethrough, to elute [$^{123}$I-IMPY. The obtained radioactivity was 41-57 MBq immediately after the synthesis. Further, the TLC analysis was conducted under the same conditions as described in Example 2, and as a result, the radiochemical purity of the compound was 93.0%.

(Example 11) Measurement of binding to amyloid

Affinity of the present compounds with amyloid was examined by the following *in vitro* binding tests.

(Method)

(1) $A\beta_{1-42}$ (Wako) was dissolved in phosphate buffer (pH 7.4,- and shaken at 37°C for 72 hours, to obtain a 1 mg/mL suspension (hereinafter referred to as amyloid suspension in these Examples) of aggregated Aβ (hereinafter referred to as amyloid in this Example).

(2) According to the method described in a literature (Naiki, H., et al., Laboratory Investigation 74, p.374-383 (1996)), the amyloid suspension was subjected to qualitative experiment based on fluorescence spectrophotometric method using Thioflavin T (manufactured by Fluka) to confirm that the aggregated Aβ obtained in (1) was amyloid (measurement conditions: excitation wavelength of 446 nm, and remission wavelength of 490 nm),

(3) A solution, in ethanol of [$^{123}$I]-2-(4 - iodophenyl)-6-methoxyimidazo[1,2-a]pyridine synthesized by a method

described above in Example 3 (radioactive concentration: 27 MBq/mL) was prepared, and diluted with a 1 %: bovine serum albumin-containing phosphate buffer (pH 7.4) to prepare a solution, corresponding to 2 nmol/L as a total amount of 2-(4 -iodophenyl)-6-methoxyimidazo[1,2-a]pyridine.

(4) To each well of a 96-well microplate, 50 μL of a solution prepared above in (3) and 50 μL of a solution (amyloid concentration may be adjusted in accordance with amyloid concentration) in a sample solution) where amyloid suspension was dissolved in a 1 % bovine serum albumen-containing phosphate buffer (pH 7.4), were added to prepare a solution of amyloid (corresponding to 400 pmol as the whole amount of 2-(4 -iodophenyl)-6-methoxyimidazo[1,2-a] pyridine), at final concentrations of 25, 62.5, 250, 625, and 1000 nmol/L.

(5) The above microplate was shaken at a given rate (400 rpm) at 22°C for 3 hours. Then, a mixed solution of each well was filtered through a glass fiber filter (trade name: Mulutiscreen™-FC, manufactured by Millipore), to separate [$^{123}$I]-2-(4-iodophenyl)-6-merhoxyimidaze[1,2-a]pyridine attached to amyloid from the free [$^{123}$I]-2-(4 -iodophenyl)-6-methoxyimidazo[1,2-a]pyridine.

(6) The glass fiber filter used for filtration of the sample solution was washed with a 1 % bovine serum albumin-containing phosphate buffer (0.5 mL x 5), and then radioactivity count of the glass fiber filter was measured with an autowell gamma system (type: ARC-301B, manufactured by Aloka).

(7) Separately, a solution of 2-(4 -iodophenyl)-6-methoxyimidazo[1,2'-a]pyridine (non-iodinated form) prepared in Example 4 in methanol was used to conduct the same procedures as above in (3) to prepare 15 μmol/L of 2-(4 -iodophenyl)-6-methoxyimidazo[1,2-a]pyridine solution. 50 μL of the solution was added to each well of a 96-well microplate in which 50 μL of a solution prepared above in (3) was placed, and further 50 μL of a solution (amyloid concentration may be adjusted in accordance with amyloid concentration in a sample solution) where amyloid suspension was dissolved in a 1% bovine serum albumin-containing phosphate buffer (pH 7.4) and 100 μL of a 1% bovine serum albumin-containing phosphate buffer (pH 7.4), were added to prepare a solution of amyloid at final concentrations of 25, 62.5, 250, 625, and 1000 nmol/L. Each microplate was subjected the same procedures as above in (5) and (6) to measure the radioactivity count remained in the glass fiber filter (hereinafter, referred to as B).

(8) Using the value of radioactive count determined in (6) and (7), the radioactive count of [$^{125}$I]-2-(4 - iodophenyl)-6-methoxyimidazo[1,2-a]pyridine specifically attached to amyloid as calculated with the following equation (1), and evaluated on the relation with the addiction amount of amyloid.

$$\text{Radioactive count} = A-B \cdots (1)$$

A relation between the radioactive count calculates above in (8) and the concentration of amyloid in the sample solution is shown in Fig. 5. As shown in the figure, a value of the radioactive count calculated above in (8) shows a tendency of an increase proportionally to the concentration of amyloid. On the conditions of the present experiment, amyloid and the compounds attached to amyloid are retained in the glass fiber. Also, a radioactivity remained on the glass fiber having no relation with amyloid was substantially removed from the value of the radioactive count calculated above in (8). Therefore, the radioactive count on the glass fiber measured in the present Examples is a value reflecting the amount of the compound specifically attached to amyloid. Thus, the fact that the value of the radioactive count determined above in (8) was increased with increase of the concentration of amyloid is a result of strongly suggesting that [$^{123}$I]-2-(4 -iodophenyl)-6-methoxylmidazo[1,2-a]pyridine is a compound having a property of binding to amyloid.
The above-mentioned results have implied that [$^{125}$I]-2-(4 -iodophenyl)-6-methoxyimidazo[1,2-a]pyridine is highly capable of binding to amyloid.

(Example I-15, Examples II-8 to II-10, Comparative Example I-6) measurement of partition coefficient based on the octanol extraction method

Partition coefficients based on the octanol extraction method (hereinafter referred to as $\log P_{octanol}$) were measured, which are generally known as an indicator of permeability of compounds through the blood-brain barrier (hereinafter referred to as BBB).

A diethyl ether solution of Compound 5 prepared in Example I-2 (Example I-15), a diethyl ether solution of compounds 9 prepared in Example II-5 (Examples II-8), a diethyl ether solution of Compound 10 prepared in Example II-6 (Example II-9), a diethyl ether solution of Compound 11 prepared in Example II-7 (Example II-10) and a diethyl ether solution of [$^{123}$I]-IMPY prepared in Reference Example 1 (Comparative Example 1-6) were each diluted with 10 mg/mL ascorbic acid-containing physiological saline solution, and adjusted to radioactive concentration of 20-30 MBq/mL. 10 μL each of the prepared sample solution was respectively added to 2 mL of octanol, further, 2 mL of 10 mmol/L phosphate buffer (pH 7.4) was added, and stirred for 30 seconds. After the mixture was centrifuged with a low-speed centrifuge (2000 rpm x 60 min.), the octanol layer and the water layer were samples each in an amount of 1 mL, and subjected to measurement of radioactivity count with an autowell gamma system (Type: ARC-301B, manufactured by Aloka). Using

the obtained radioactivity count, $logP_{octanol}$ was calculated in accordance with the equation (2).

$$\log P_{octanol} = \log_{10}\left(\frac{\text{Radioactivity count of octanol layer}}{\text{Radioactivity count of water layer}}\right) \cdots (2)$$

The results are shown in Table 1. As shown in the table, all compounds showed a $logP_{octanol}$ value between 1 and 3. It is known that compounds permeable to BBB show a $logP_{octanol}$ value between 1 and 3 (Douglas D. Dischino et al., J. Nucl. Med., (1983), 24, p.1030-1038). Thus, it is implied that all compounds have a BBB permeability comparable to IMPY.

Table 1: $logP_{octanol}$ value of the present compound

| Experiment | Compound | $logP_{octanol}$ value |
|---|---|---|
| Comparative Example 1 | [$^{123}$I]-IMPY | 1.9 |
| Example 12 | [$^{123}$I]-6-iodo-2-(4-methoxyphenyl) imidazo[1,2-a]pyridine | 1.7 |
| Example 13 | [$^{123}$I]-2-(4 -iodophenyL)-6-methoxyimidazo[1,2-a] pyridine | 2.3 |
| Example 14 | [$^{123}$I]-2-(4 -fluorophenyl)-6-iodoimidazo[1,2-a] pyridine | 2.3 |

(Examples 15-16, Comparative Example 2) measurement of transferability into brain and clearance

Using [$^{123}$I]-2-(4 -iodophenyl)-6-methoxyimidazo[1,2-a]pyridine and [$^{123}$I]-2-(4 -fluorophenyl)-6-iodoimidazo [1,2-a]pyridine, a time course change of radioactive accumulation in brain of male Wistar rats (7-week old) was measured.

(Method)

0.05 mL (20-30 MBq/mL in radioactive concentration) of a solution of [$^{123}$I]-2-(4 -iodophenyl)-6--methoxyimidazo [1,2-a]pyridine (Example 15) prepared above in Example 2, and [$^{123}$I]-2-(4 -fluorophenyl)-6-iodoimidazo[1,2-a]pyridine (Examples 16) prepared above in Example 10 in a 10 mg/mL ascorbic acid-containing physiological saline solution was injected under thiopental anesthesia into the tail vein of respective Wistar rats (7-week old). The rats were sacrificed by bleeding from abdominal artery, and brains were removed and subjected to measurement of mass of brains (hereinafter referred to as A in this Example) and further subjected to measurement of radioactivity with a single channel analyzer (detector type: SP-20 manufactured by OHYO KOKEN KOGYO Co., Ltd.) 2, 5, 30 and 60 minutes after the injection. Also, radioactivity (hereinafter referred to as B in this Examples) of the rest of the whole body was measured in the same manner as above. Using these measurement results, radioactive accumulation per unit weight of brain (%ID/g) at the respective time points were calculated in accordance with the following formula (3) (Examples 15 and 16).
Separately, a solution of [$^{123}$I]-IMPY in a 10 mg/mL ascorbic acid-containing physiological saline solution (20-30 MBq/mL in radioactive concentration) was prepared. The same procedures as above was conducted and subjected to the same procedures as above to calculated radioactive accumulation per unit weight of a brain (%ID/g) at the respective time points were calculated.
Three animals were used for Example 15, Example 16 and Comparative Example 2 at the respective time points.

$$\%ID/g = \frac{\mathrm{A}}{\mathrm{B} \times 1000 \times brain\ weight} \times 100 \quad \cdots \quad (3)$$

The results are shown in Table 2. As shown in Table 2, [$^{123}$I-2-(4 -iodophenyl)-6-methoxyimidazo[1,2-a]pyridine (Example 15) and [$^{123}$I]-2-(4 -fluorophenyl)-6-iodoimidazo[1,2-a]pyridine (Example 16) showed a accumulation comparable to $^{123}$I-IMPY (Comparative Example 2) at the time point of two minutes after the injection, and then showed a tendency to rapidly clear away in 60 minutes. These results suggest that [$^{123}$I]-2-(4-iodo-phenyl)-6-methoxyimidazo[1,2-a]pyridine and [$^{123}$I]-2-(4 -fluorophenyl)-6-iodoimidazo[1,2-a]pyridine possess excellent transferability to brain and rapid

clearance from brain like $^{123}I$-IMPY.

Table 2: Radioactive accumulation in brain of the present compound after intravenous injection (rats)

| Compound | Radioactive accumulation per unit weight (%ID/g) | | | |
|---|---|---|---|---|
| | After 2 min. | After 5 min. | After 30 min. | After 60 min. |
| Example 15 | 1.26 | 0.89 | 0.19 | 0.09 |
| Example 16 | 1.00 | 0.72 | 0.12 | 0.05 |
| Comparative Example 3 | 1.19 | 0.97 | 0.23 | 0.09 |

(Example 17) Confirmation of imaging of amyloid in brain with [$^{123}I$]-6-iodo-2-(4 -methoxyphenyl)imidazo[1,2-a]pyridine

The following experiment was carried out in order to examine whether amyloid in brain can be imaged by the compound of the present invention.

(1) $A\beta_{1-42}$ (Wako) was dissolved in phosphate buffer-(pH 7.4) and shaken at 37 °C for 72 hours, to obtain a 1 mg/mL suspension of aggregated Aβ (hereinafter referred to as amyloid suspension in this Example).

(2) Under thiopental anesthesia, 2.5 μL (corresponding to 25 μg) of the amyloid suspension was injected into an amygdaloid nucleus on one side of a male Wistar rat (7-week old). As a control, 2.5 μL of a phosphate buffered physiological saline solution (pH 7.4) was injected into an amygdaloid nucleus on the other side of the rat. The rats were examined 1 day after the injection of the amyloid suspension and the phosphate buffered physiological saline solution, (pH 7.4).

(3) [$^{123}I$]-6-iodo-2-(4 -methoxyphenyl)imidazo[1,2-a]pyridine was dissolved in a 10 mg/mL ascorbic acid-containing physiological saline solution, to obtain a sample solution (32 MBq/mL in radioactivity concentration). This sample solution, was injected under thiopental anesthesia into the rat through the tail vein (dosage: 0.5 mL, dosed radioactivity: 16 MBq equivalent).

(4) Brain was removed 60 minutes, after the injection to prepare a brain slice of 10 μm in thickness with a microtome (type: CM3050S, manufactured by LEICA). The brain slice was exposed to an imaging plate for 20 hours, and then image analysis was carried out by use of a Biro-imaging Analyser (type: BAS-2500; manufactured by FUJIFILM Corporation).

(5) After the completion of the image analysis using the Bio-imaging Analyzer, pathological staining with Thioflavin T was carried out to perform imaging by use of a fluorescence microscope (manufactured by NIKON Corporation; type: TE2000-U model; excitation wavelength: 400-440 nm; detection wavelength: 470 nm). Thus, it was confirmed that amyloid was deposited on the slice (Fig. 8b).

(Result)

Fig. 8 shows images by autoradiogram and Thioflavin T staining of the brain slice of the rat to which amyloid was injected intracerebrally. As shown in this figure, a marked accumulation of radioactivity in the specimen was observed in the amygdaloid nucleus on the side to which the amyloid suspension was injected. From the result of Thioflavin T staining in the site where radioactivity is accumulated, it was confirmed that amyloid was present in the site. On the other hand, no significant accumulation of radioactivity was observed in the amygdaloid nucleus on the side to which the physiological saline solution was injected, compared with the other sites.

These results imply that [$^{123}I$]-6-iodo-2-(4 - methoxyphenyl)imidazo[1,2-a]pyridine possesses a property of accumulating on intracerebral amyloid and a capability of imaging intracerebral amyloid.

(Example 18) Confirmation of imaging of amyloid in brain with [$^{123}I$]-2-(4 -iodophenyl)-6-methoxyimidazo[1,2-a]pyridine

The following experiment was carried out in order to examine whether amyloid in brain can be imaged by the compound of the present invention.

(Method)

The same procedures as in Example 17 was carried out except that [$^{123}I$]-2-(4 -iodophenyl)-6-methoxyimidazo [1,2-a]pyridine was dissolved in a 10 mg/mL ascorbic acid-containing physiological saline solution (20 MBq/mL in radioactive concentration) to obtain a sample solution, and thus a capability of imaging intracerebral amyloid was confirmed.

(Result)

Fig. 9 shows images by autoradiogram and Thioflavin T staining of the brain slice of the rat to which amyloid was injected intracerebrally. As shown in this figure, a marked accumulation of radioactivity in the specimen was observed in the amygdaloid nucleus on the side to which the amyloid suspension was injected. From the result of Thioflavin T staining in the site where radioactivity is accumulated, it was confirmed that amyloid was present in the site. On the other hand, no significant accumulation of radioactivity was observed in the amygdaloid nucleus on the side to which the physiological saline solution was injected, compared with the other sites. These results imply that [$^{121}$I]-2-(4 -iodophenyl)-6-methoxyimidazo[1,2-a]pyridine possesses a property of accumulating on intracerebral amyloid and a capability of imaging intracerebral amyloid.

(Example 19) [$^{123}$I]-2-(4 -fluorophenyl)-6-iodoimidazo[1,2-a]pyridine

The following experiment was carried out in order to examine whether amyloid in brain can be imaged by the compound of the present invention.

(Method)

The same procedures as in Example 17 was carried out except that [$^{123}$I]-2-(4 -fluorophenyl)-6-iodoimidazo [1,2-a]pyridine was dissolved in a 10 mg/mL ascorbic acid-containing physiological saline solution (20 MBq/mL in radioactive concentration) to obtain a sample solution, and thus a capability of imaging intracerebral amyloid was confirmed.

(Result)

Fig. 10 shows images by autoradiogram and Thioflavin T staining of the brain slice of the rat to which amyloid was injected intracerebrally. As shown in this figure, a marked accumulation of radioactivity in the specimen was observed in the amygdaloid nucleus on the side to which the amyloid suspension was injected. From the result of Thioflavin T staining in the site where radioactivity is accumulated, it was confirmed that amyloid was present in the site. On the other hand, no significant accumulation of radioactivity was observed in the amygdaloid nucleus on the side to which the physiological saline solution was injected, compared with the other sites. These results imply that [$^{123}$I]-2-(4 -fluorophenyl)-6-iodoimidazo[1,2-a]pyridine possesses a property of accumulating on intracerebral amyloid and a capability of imaging intracerebral amyloid.

(Example 20) Measurement of transferability into brain and clearance

Using [$^{123}$I]-6-iodo-2-(4 -methoxyphenyl)imidazo[1,2-a]pyridine, a time course change of radioactive accumulation in brain of male Wistar rats (7-week old) was measured.

(Method)

A time course change of radioactive accumulation in brain of the above rats was measured by conducting the same procedures as in Examples 15-16 except that [$^{123}$I]-6-indo-2-(4 -methoxyphenyl)imidazo[1,2-a]pyridine in a 10 mg/mL ascorbic acid-containing physiological saline solution (20-30 MBq/mL in radioactive concentration) was used.

The results are shown in Table 3. As shown in Table 3, [$^{123}$I]-6-iodo-2-(4 -methoxyphenyl)imidazo[1,2-a]pyridine showed a accumulation comparable to $^{123}$I-IMPY (refer to the above Comparative Example 2) at the time point of two minutes after the injection, and then showed a tendency to rapidly clear away in 60 minutes. These results suggest that [$^{123}$I]-6-iodo-2-(4 - methoxyphenyl)imidazo[1,2-a]pyridine possesses excellent transferability to brain and rapid clearance from brain like $^{123}$I-IMPY.

Table 3: Radioactive accumulation in brain of the present compound after intravenous injection (rats)

| Compound | Radioactive accumulation per unit weight (%ID/g) | | | |
|---|---|---|---|---|
| | After 2 min. | After 5 min. | After 30 min. | After 60 min. |
| Example 20 | 1.04 | 0.72 | 0.12 | 0.04 |

(Example 21) Measurement of radioactive distribution ratio in each organ with [$^{123}$I]-2-(4 -iodophenyl)-6-methoxyimidazo[1,2-a]pyridine

In order to confirm that a compound of the present invention can be distributed in a target organ and has good clearance to the outside of the body, [$^{123}$I]-2-(4 - iodophenyl)-6-methoxyimidazo[1,2-a]pyridine was used to measure a time-course change of radioactive accumulation to each organ in SD rat (8 week).

To 100 μL of a solution, of 2-(4 - tributylstannylphenyl)-6-methoxyimidazo[1,2-a]pyridine in acetonitrile (concentration: 1 mg/mL), 100 μL of 1 mol/L sulfuric acid, 10 μL of 1 mmol/L sodium iodide, 60 μL of [$^{123}$I]sodium iodine of 728 MBq, and 10 μL of 30% (w/v) hydrogen peroxide were added. After the mixed solution was left to stand at 40°C for 10 minutes, the solution was subjected to HPLC under the following conditions, to obtain [$^{123}$I]-2-(4 -iodophenyl)-6-methoxyimidazo[1,2-a]pyridine as a fraction.

HPLC conditions:

Column: YMC-Pack Pro C8 (trade name; manufactured by YMC;
size: 4.6 x 150 mm)
Mobile phase: 10 mM formic acid (pH 3.0)/acetonitrile = 80/20 to 10/90 (0 minute to 30 minutes)
Flow rate: 1.0 mL/min.
Defector: Ultraviolet visible absorptiometer (Detection
wavelength: 254 nm) and radioactivity counter (manufactured by raytest: type STEFFI)

10 ml of water was added to the fraction. The resulting solution was passed through a Sep-Pak C18 column (trade name: Sep-Pak (registered trademark) Light C18 Cartridges manufactured by Waters: the packed amount of the packing agent: 130 mg) so that the column adsorbs and collects [$^{123}$I]-2-(4 -iodophenyl)-6-methoxyimidazo[1,2-a]pyridine. The column was rinsed with 1 mL of water, and then 1 mL of diethylether was passed therethrough to elute[$^{123}$I]-2-(4 -iodophenyl)-6-methoxyimidazo[1,2-a]pyridine. The amount of radioactivity of the obtained compound was 448 MBq at the end of the synthesis. Further, the TLC analysis was conducted under the following conditions, and as a result, the radiochemical purity of the compound was 98%.

TLC analysis conditions:

TLC plate: Silica Gel 60 $F_{254}$ (trade name; manufactured by Merck & Co., Inc.)
Mobile phase: ethyl acetate/methanol/diethylamine = 100/4/1
Detector: Rita Star (trade name; manufactured by raytest)

A solution of [$^{123}$I]-2-(4 -iodophenyl)-6-methoxyimidazo[1,2-a]pyridine in diethylether was diluted with a 10 mg/mL ascorbic acid-containing physiological saline solution and adjusted to 8-12 MBq/mL in radioactive concentration. 0.2 mL each of the prepared sample solution was administered under non-thiopental anesthesia into the tail vein of the above rat. The rats were sacrificed by bleeding from abdominal artery, and each organ shown in Table 4 was removed 5, 30, 60 and 180 minutes after the administration. Each removed organ was subjected to measurement of mass and radioactivity witch the same method as in Example 15. Also, radioactivity of the whole body of the rat after removal of the organs (hereinafter, refers to rest of the whole body) was measured. Using these measurement results, radioactive distribution per unit weight of each organ (%ID/g) at the respective time points was calculated in accordance with the following formula (4).
Three animals were used for the experiment at the respective time points.

$$\%ID/g = \frac{R^T}{(R^S + R^R) \times M^T} \times 100 \cdots (6)$$

$R^T$: Radioactivity of target organ (cpm)
$R^S$: Sum of radioactivity of all the organs (cpm)
$R^R$: Radioactivity of the rest of the whole body (cpm)
$M^T$: Mass of target organ (g)

The results are shown in Table 4. As shown in Table 4 , [$^{123}$I]-2-(4 -iodophenyl)-6-methoxyimidazo[1,2-a]pyridine was distributed to each organ at the time point of 5 minutes after the administration, and then most of the radioactivity was distributed to small intestine and large intestine. In addition, radioactive distribution thereof was transferred from small intestine to large intestine. Thus, [$^{123}$I]-2-(4 -iodophenyl)-6-methoxyimidazo[1,2-a]pyridine was found to be biliary-excreted rapidly after the administration and have a good clearance to the outside of the body.
Also, observing brain, heart, lung, pancreas and bone in which amyloid is considered to accumulate, obvious radioactive accumulation was found in all these organs at the time point of 5 minutes after the administration, and thus distribution of a compound of Example 13 was confirmed. Moreover, the ratio of the time point of 5 minutes after the administration to the time point of 180 minutes after the administration ((%ID/g at the time point of 5 minutes after the administration)

/ (%ID/g at the time point of 180 minutes after the administration)) showed high values such as 43 for brain, 12 for heart, 7 for lung, 8 for pancreas and 3 for bone. Thus, it has been shown that rapid radioactive distribution and rapid clearance are performed in organs in which amyloid is considered to accumulate.
From the above, it has been shown that a radioactive distribution at an early stage after administration and a rapid clearance to the outside of the body, which are required for an amyloid detecting reagent in a biological tissue, have been attained.

Table 4

| Tissue/ organ | 5 min. after administration | | 30 min. after administration | | 60 min. after administration | | 180 min. after administration | |
|---|---|---|---|---|---|---|---|---|
| | Average | Standard deviation | Average | Standard deviation | Average | Standard deviation | Average | Standard deviation |
| Blood | 0.290 | 0.015 | 0.240 | 0.007 | 0.203 | 0.017 | 0.139 | 0.018 |
| Brain | 1.175 | 0.172 | 0.220 | 0.030 | 0.111 | 0.056 | 0.027 | 0.007 |
| Heart | 0.752 | 0.019 | 0.220 | 0.021 | 0.134 | 0.018 | 0.061 | 0.009 |
| Lung | 1.109 | 0.240 | 0.410 | 0.026 | 0.243 | 0.013 | 0.163 | 0.026 |
| Liver | 1.857 | 0.205 | 1.197 | 0.129 | 0.836 | 0.071 | 0.374 | 0.028 |
| Spleen | 0.460 | 0.071 | 0.207 | 0.032 | 0.154 | 0.038 | 0.084 | 0.003 |
| Pancreas | 0.709 | 0.092 | 0.338 | 0.051 | 0.247 | 0.028 | 0.088 | 0.013 |
| Stomach | 0.203 | 0.067 | 1.081 | 0.041 | 1.476 | 0.847 | 1.272 | 0.337 |
| Small intestine | 0.729 | 0.110 | 3.964 | 0.277 | 5.924 | 0.741 | 2.870 | 0.227 |
| Large intestine | 0.122 | 0.007 | 0.096 | 0.005 | 0.099 | 0.008 | 3.294 | 0.172 |
| Kidney | 1.434 | 0.070 | 2.102 | 0.255 | 1.630 | 0.100 | 0.643 | 0.080 |
| Adrenal gland | 5.163 | 1.489 | 2.364 | 0.220 | 1.775 | 0.167 | 0.754 | 0.242 |
| Bone | 0.223 | 0.027 | 0.136 | 0.014 | 0.102 | 0.014 | 0.070 | 0.010 |
| Marrow | 0.937 | 0.076 | 0.285 | 0.073 | 0.000 | 0.000 | 0.000 | 0.000 |
| Muscle | 0.344 | 0.015 | 0.092 | 0.006 | 0.062 | 0.010 | 0.027 | 0.002 |

## INDUSTRIAL APPLICABILITY

The reagent for detecting amyloid in biological tissues according to the present invention can be utilized as diagnostic agents for amyloid protein *in vitro* and *in vivo* in amyloidosis such as systemic amyloidosis.

## BREIF DESCRIPTION OF THE DRAWINGS

Fig. 1 is a scheme of synthesis of 2-(4 - tributylstannylphenyl)-6-methoxyimidazo[1,2-a]pyridine.
Fig. 2 is a scheme of synthesis of 2-(4 -iodophenyl)-6-methoxylmidazo[1,2-a]pyridine.
Fig. 3 is a scheme of synthesis of 6-indo-2-(4 - methoxyphenyl)imidazo[1,2-a]pyridine.
Fig. 4 is a scheme of synthesis of 6-tributylstannyl-2-(4 -methoxyphenyl)imidazo[1,2-a]pyridine.
Fig. 5 is a scheme of synthesis of 2-(4 -fluorophenyl)-6-iodoimidazo[1,2-a]pyridine.
Fig. 6 is a scheme of synthesis of 6-tributylstannyl-2-(4 -fluorophenyl)imidazo[1,2-a]pyridine.
Fig. 7 is a relationship between amyloid concentration and radioactive concentration in sample solutions.
Fig. 8(a) is an autoradiogram of the brain slice after the injection of [$^{123}$I]-6-iodo-2-(4 - methoxyphenyl)imidazo[1,2-a]pyridine, and Fig. 8(b) is a fluorescent microscopic image of the Thioflavin T stained sample (a magnification of

the site to which the amyloid suspension was injected).

Fig. 9(a) is an autoradiogram of the brain slice after the injection of [123-2-(4 -iodophenyl)-6-methoxyimidazo[1,2-a] pyridine, and Fig. 9(b) is a fluorescent microscopic image of the Thioflavin T stained sample (a magnification of the site to which the amyloid suspension was injected).

Fig. 10(a) is an autoradiogram of the brain slice after the injection of [$^{123}$I]-2-(4 -fluorophenyl)-6-iodoimidazo[1,2-a] pyridine, and Fig. 10(b) is a fluorescent microscopic image of the Thioflavin T stained sample (a magnification of the site to which the amyloid suspension was injected).

## Claims

1. A reagent for detecting amyloid deposited in a biological tissue, which comprises a compound represented by the following formula (1), or a salt thereof:

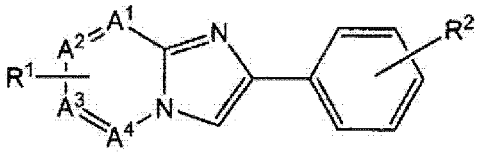

(1)

wherein $A^1$, $A^2$, $A^3$ and $A^4$ independently represent a carbon or nitrogen,
$R^1$ is a group selected from the group consisting of a hydrogen, hydroxyl group, carboxyl group, sulfuric acid group, amino group, nitro group, cyano group, non-radioactive halogen substituent, alkyl substituent with 1 to 4 carbon atoms and alkoxy substituent, with 1 to 4 carbon atoms, and
$R^2$ is a radioactive halogen substituent,
provided that at least one of $A^1$, $A^2$, $A^3$ and $A^4$ represents a carbon, and $R^1$ binds to a carbon represented by $A^1$, $A^2$, $A^3$ or $A^4$.

2. A reagent according to claim 1, wherein at least three of $A^1$, $A^2$, $A^3$ and $A^4$ represent carbons.

3. A reagent according to claim 2, wherein all of $A^1$, $A^2$, $A^3$ and $A^4$ represent carbons.

4. A reagent according to any one of claims 1 to 3, wherein $R^2$ is selected from the group consisting of $^{18}F$, $^{75}Br$, $^{76}Br$, $^{123}I$, $^{124}I$, $^{125}I$ and $^{131}I$.

5. A reagent according to any one of claims 1 to 4, wherein the biological tissue is brain, heart, lung, pancreas, bone, or joint.

6. A reagent for detecting amyloid deposited in a biological tissue, which comprises a compound represented by the following formula (1), or a salt thereof:

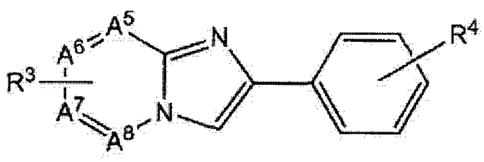

(2)

wherein $A^5$, $A^6$, $A^7$ and $A^8$ independently represent a carbon or nitrogen,
$R^3$ is a radioactive halogen substituent, and
$R^4$ is a group selected from the group consisting of a hydrogen, carboxyl group, sulfuric acid group, nitro group, cyano group, non-radioactive halogen substituent, alkyl substituent, with 1 to 4 carbon atoms and methoxy substituent,
provided that at least one of $A^5$, $A^6$, $A^7$ and $A^8$ represents a carbon, and $R^3$ binds to a carbon represented by $A^5$, $A^6$, $A^7$ or $A^8$.

**7.** A reagent according two claim 6, wherein at least three of $A^5$, $A^6$, $A^7$ and $A^8$ represent carbons .

**8.** A reagent according to claim 7, wherein all of $A^5$, $A^6$, $A^7$ and $A^8$ represent carbons.

**9.** A reagent according to any one of claims 6 to 8, wherein $R^2$ is selected from the group consisting of $^{18}F$, $^{75}Br$, $^{76}Br$, $^{123}I$, $^{124}I$, $^{125}I$ and $^{131}I$.

**10.** A reagent according to any one of claims 6 to 9, wherein the biological tissue is brain, heart, lung, pancreas, bone, or joint.

**11.** A process for production of a radioactive halogen-labeled organic compound, which comprises a step of preparing a reaction solution containing, together with a radioactive halogen ion, a compound represented by the following formula (3) or a salt thereof:

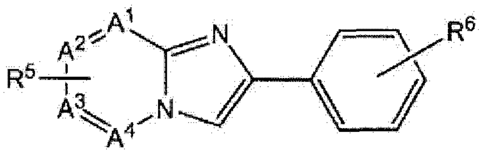

(3)

wherein $A^1$, $A^2$, $A^3$ and $A^4$ independently represent a carbon or nitrogen,
$R^5$ is a group selected from the group consisting of a hydrogen, hydroxyl group, carboxyl group, sulfuric acid group, amino group, nitro group, cyano group, non-radioactive halogen substituent, alkyl substituent, with 1 to 4 carbon atoms and alkoxy substituent with 1 to 4 carbon atoms, and
$R^6$ is a group selected from the group consisting of a non-radioactive halogen substituent, nitro group, trialkylstannyl substituent having alkyl chains with from 1 to 4 carbon atoms, triphenylstannyl group and trialkylammonium group having alkyl chains with from 1 to 4 carbon atoms,
provided that at least one of $A^1$, $A^2$, $A^3$ and $A^4$ represents a carbon, and $R^5$ binds to a carbon represented by $A^1$, $A^2$, $A^3$ or $A^4$, and

a step of giving a reaction condition to the reaction solution to synthesize a compound represented by the following formula (1) :

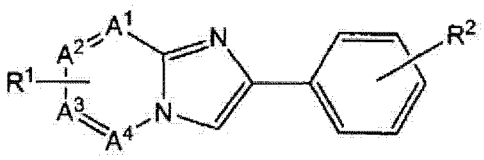

(1)

wherein $A^1$, $A^2$, $A^3$ and $A^4$ independently represent a carbon or nitrogen,
$R^1$ is a group selected from the group consisting of a hydrogen, hydroxyl group, carboxyl group, sulfuric acid group, amino group, nitro group, cyano group, non-radioactive halogen substituent, alkyl substituent with 1 to 4 carbon atoms and alkoxy substituent with 1 to 4 carbon atoms, and
$R^2$ is a halogen substituent,
provided that at least one of $A^1$, $A^2$, $A^3$ and $A^4$ represents a carbon, and $R^1$ binds to a carbon represented by $A^1$, $A^2$, $A^3$ or $A^4$.

or a salt thereof.

**12.** A process for production of a radioactive halogen-labeled organic compound, according to claim 11, wherein at least three of $A^1$, $A^2$, $A^3$ and $A^4$ represent carbons.

**13.** A process for production; of a radioactive halogen labeled organic compound, according to claim 12, wherein, all of $A^1$, $A^2$, $A^3$ and $A^4$ represent carbons.

**14.** A process for production of a radioactive halogen-labeled organic compound, according to any one of claims 11 to 13, wherein $R^6$ is a group selected from the group consisting of a non-radioactive iodine, trialkylstannyl substituent having alkyl chains with from 1 to 4 carbon atoms and triphenylstannyl substituent,
a radioactive halogen ion is selected from the group, consisting of $^{123}I$ ion, $^{124}I$ ion, $^{125}I$ ion and $^{131}I$ ion, and $R^2$ is selected from the group consisting of $^{123}I$, $^{124}I$, $^{125}I$ and $^{131}I$.

**15.** A process for production of a radioactive halogen-labeled organic compound, according to any one of claims 11 to 13, wherein $R^6$ is a nitro substituent or trialkylammonium group having alkyl chains with from 1 to 4 carbon atoms, a radioactive halogen ion is $^{18}F$ ion, and
$R^2$ is $^{18}F$.

**16.** A process for production of a radioactive halogen-labeled organic compound, according to any one of claims 11 to 13, wherein $R^6$ is a non-radioactive bromine, the radioactive halogen ion is $^{75}Br$ ion or $^{76}Br$ ion, and $R^2$ is $^{75}Br$ or $^{76}Br$.

**17.** A process for production of a radioactive halogen-labeled organic compound, which comprises a step of preparing a reaction solution containing, together with a radioactive halogen ion, a compound represented by the following formula (4) or a salt thereof:

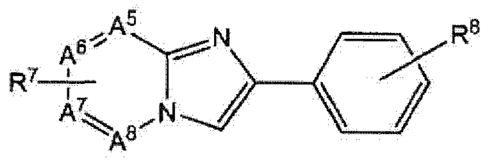

(4)

wherein $A^5$, $A^6$, $A^7$ and $A^2$ independently represents a carbon or nitrogen,
$R^7$ is a group selected from the group consisting of a non-radioactive halogen substituent, nitro group, trialkylstannyl substituent having alkyl chains with from 1 to 4 carbon atoms, triphenylstannyl group and trialkylammonium group having alkyl chains with from 1 to 4 carbon atoms, and
$R^8$ is a group selected from the group consisting of a hydrogen, carboxyl group, sulfuric acid group, nitro group, cyano group, non-radioactive halogen substituent, alkyl substituent with 1 to 4 carbon atoms and methoxy substituent,
provided that at least one of $A^5$, $A^6$, $A^7$ and $A^8$ represents a carbon, and $R^7$ binds to a carbon represented by $A^5$, $A^6$, $A^7$ or $A^8$, and

a step or giving a reaction condition to the reaction solution to synthesize a compound represented by the following formula (2):

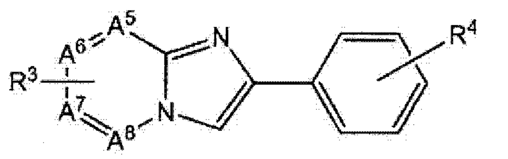

(2)

wherein $A^5$, $A^6$, $A^7$ and $A^8$ independently represent a carbon or nitrogen,
$R^3$ is a radioactive halogen substituent, and
$R^4$ is a group selected from the group consisting of a hydrogen, carboxyl group, sulfuric acid group, nitro group, cyano group, non-radioactive halogen substituent, alkyl substituent with 1 to 4 carbon atoms and methoxy substituent,
provided that at least one of $A^5$, $A^6$, $A^7$ and $A^8$ represents a carbon, and $R^3$ binds to a carbon represented by $A^5$, $A^6$, $A^7$ or $A^8$,

or a salt thereof.

**18.** A process for production of a radioactive halogen-labeled organic compound, according to claim 17, wherein, at

least three of $A^5$, $A^6$, $A^7$ and $A^8$ represent carbons.

**19.** A process for production of a radioactive halogen labeled organic compound, according to claim 18, wherein, all of $A^5$, $A^6$, $A^7$ and $A^8$ represent carbons.

**20.** A process for production of a radioactive halogen-labeled organic compound, according to any one of claims 17 to 19, wherein $R^7$ is a group selected from the group, consisting of a non-radioactive iodine, trialkylstannyl substituent having alkyl chains with from 1 to 4 carbon atoms and triphenylstannyl substituent, the radioactive halogen ion is selected from the group consisting of $^{123}I$ ion, $^{124}I$ ion, $^{125}I$, ion and $^{131}I$ ion, and $R^3$ is selected from the group consisting of $^{123}I$, $^{124}I$, $^{125}I$, and $^{131}I$.

**21.** A process for production of a radioactive halogen-labeled organic compound, according to any one of claims 17 to 19, wherein, $R^7$ is a nitro substituent or trialkylammonium group having alkyl chains with from 1 to 4 carbon atoms, the radioactive halogen ion is $^{18}F$ ion, and
$R^3$ is $^{18}F$.

**22.** A process for production of a radioactive halogen-labeled organic compound, according to any one of claims 17 to 19, wherein $R^7$ is a non-radioactive bromide, the radioactive halogen ion is $^{75}Br$ ion or $^{76}Br$ ion, and $R^3$ is $^{75}Br$ or $^{76}Br$.

**23.** A precursor compound for preparing a radioactive halogen-labeled organic compound, which is represented by the following formula (3):

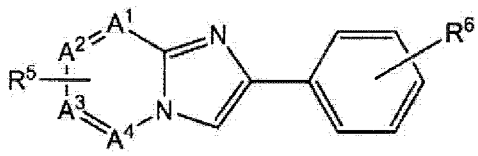

(3)

wherein $A^1$, $A^2$, $A^3$ and $A^4$ independently represent a carbon or nitrogen,
$R^5$ is a group selected from the group consisting of a hydrogen, hydroxyl group, carboxyl group, sulfuric acid group, amino group, nitro group, cyano group, non-radioactive halogen substituent, alkyl substituent with 1 to 4 carbon atoms and alkoxy substituent with 1 to 4 carbon atoms, and
$R^6$ is a group selected from the group consisting of a non-radioactive halogen substituent, nitro group, trialkylstannyl substituent having alkyl chains with from 1 to 4 carbon atoms, triphenylstannyl group and trialkylammonium group having alkyl chains with from 1 to 4 carbon atoms,
provided that at least one of $A^1$, $A^2$, $A^3$ and $A^4$ represents a carbon, and $R^5$ binds to a carbon represented by $A^1$, $A^2$, $A^3$ or $A^4$, or a salt thereof.

**24.** A precursor compound for preparing a radioactive halogen-labeled organic compound or a salt thereof, according to claim 23, wherein at least three of $A^1$, $A^2$, $A^3$ and $A^4$ represent carbons.

**25.** A precursor compound for preparing a radioactive halogen-labeled organic compound or a salt thereof, according to claim 24, wherein all of $A^1$, $A^2$, $A^3$ and $A^4$ represent carbons.

**26.** A precursor compound for preparing a radioactive halogen-labeled organic compound, which is represented by the following formula (4):

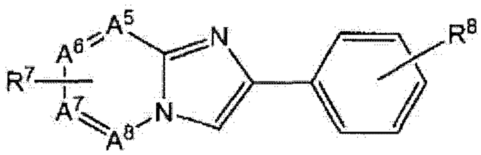

(4)

wherein $A^5$, $A^6$, $A^7$ and $A^8$ independently represent a carbon or nitrogen,

$R^7$ is a group selected from the group consisting of a non-radioactive halogen substituent, nitro group, trialkylstannyl substituent having alkyl chains with from 1 to 4 carbon atoms, triphenylstannyl group and trialkylammonium group, having alkyl chains with from 1 to 4 carbon atoms, and
$R^8$ is a group selected from the group consisting of a hydrogen, carboxyl group, sulfuric acid group, nitro group, cyano group, non-radioactive halogen substituent, alkyl substituent with 1 to 4 carbon atoms and methoxy substituent,
provided that at least one of $A^5$, $A^6$, $A^7$ and $A^8$ represents a carbon, and $R^7$ binds to a carbon represented by $A^5$, $A^6$, $A^7$ or $A^8$, or a salt thereof.

**27.** A precursor compound for preparing a radioactive halogen-labeled organic compound or a salt thereof, according to claim 26, wherein at least three of $A^5$, $A^6$, $A^7$ and $A^8$ represent carbons.

**28.** A precursor compound for preparing a radioactive halogen-labeled organic compound or a salt thereof, according to claim 27, wherein all of $A^5$, $A^6$, $A^7$ and $A^8$ represent carbons.

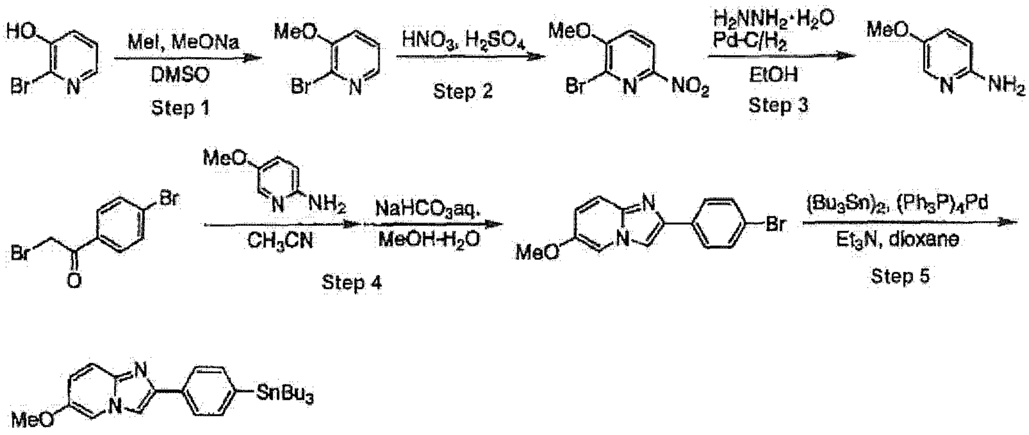


Figure 1

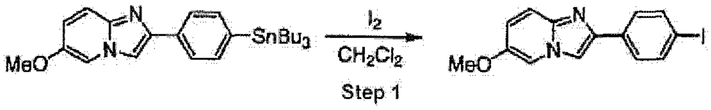


Figure 2

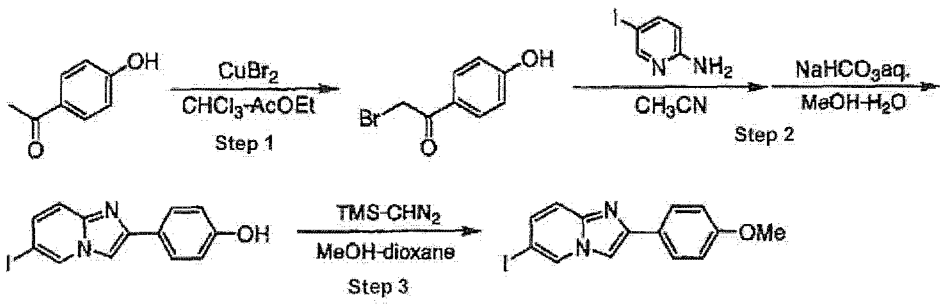


Figure 3

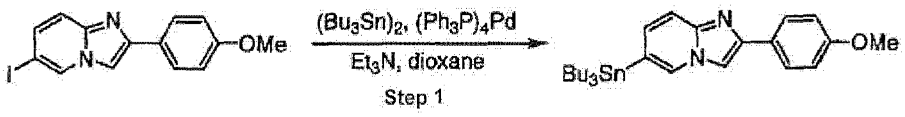


Figure 4

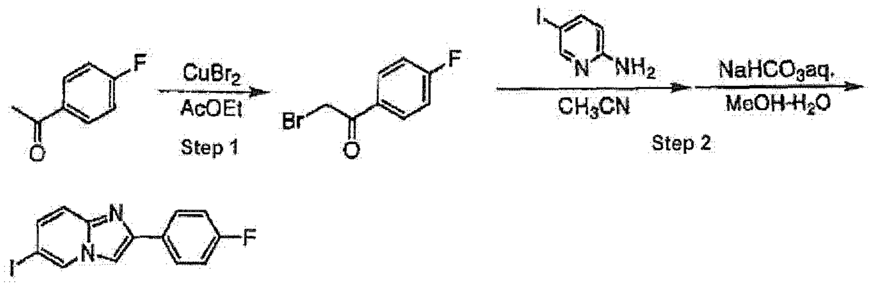


Figure 5

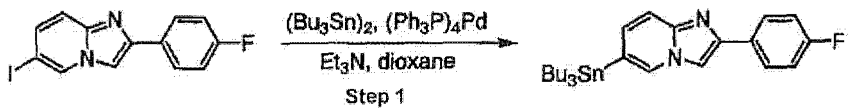


Figure 6

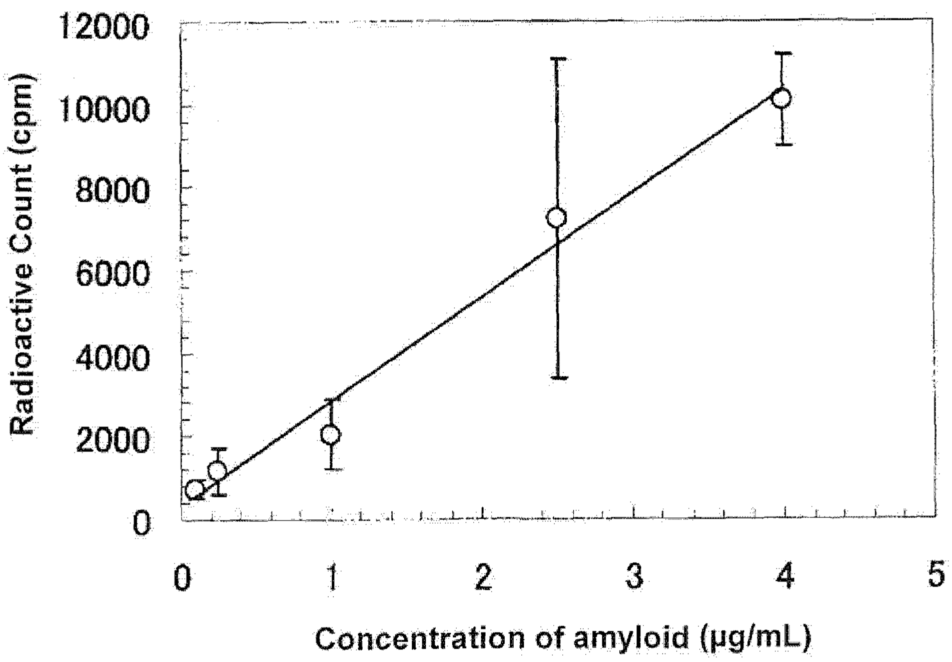


Figure 7

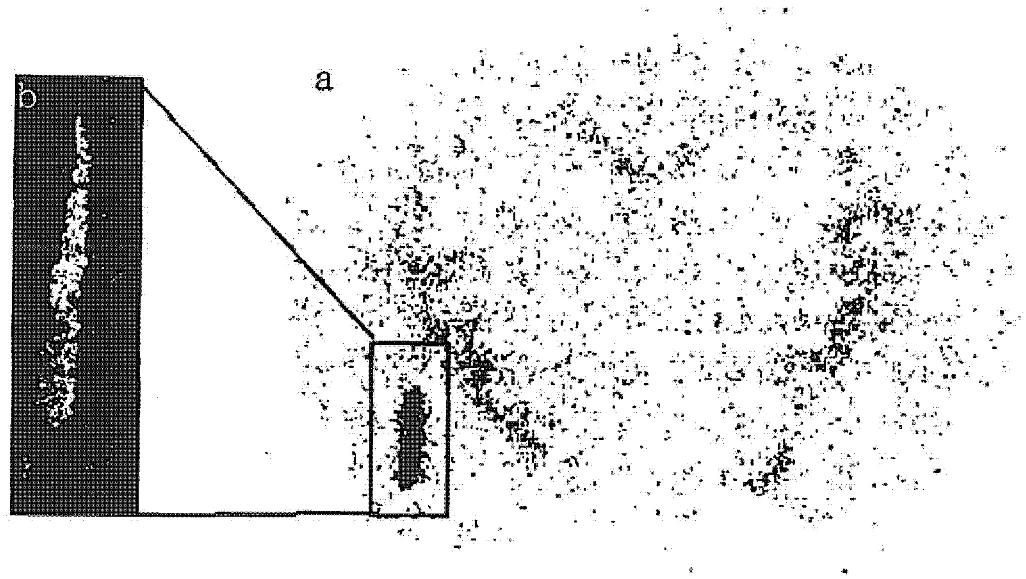


Figure 8

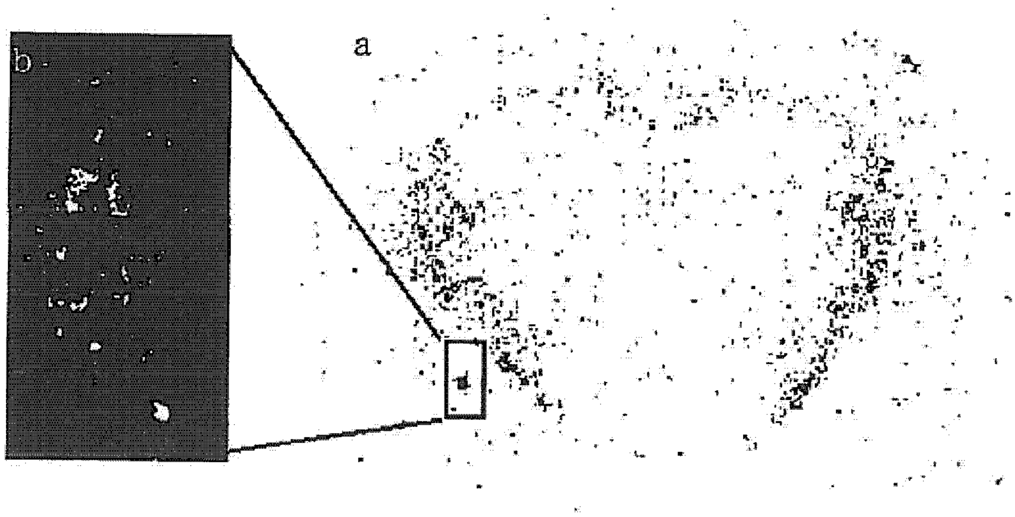


Figure 9

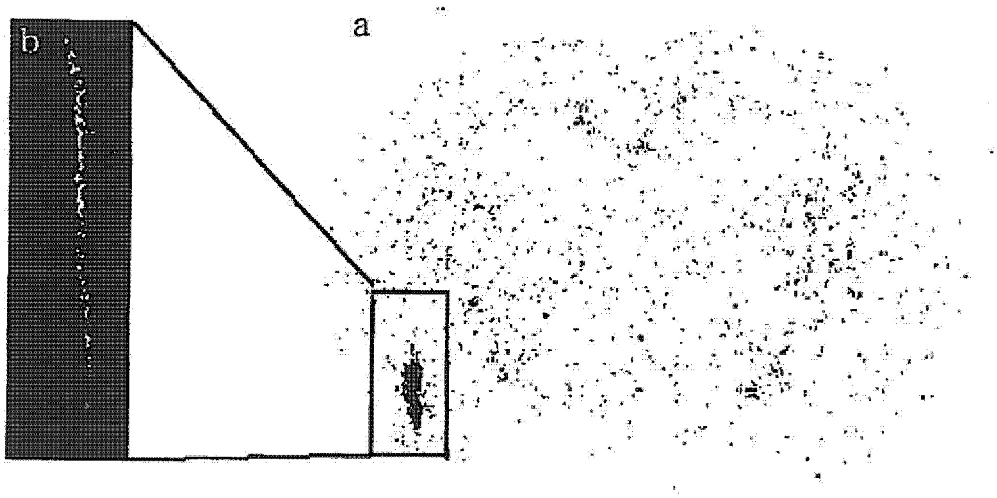


Figure 10

## INTERNATIONAL SEARCH REPORT

International application No.
PCT/JP2008/069503

A. CLASSIFICATION OF SUBJECT MATTER
*A61K51/00*(2006.01)i, *C07D471/04*(2006.01)i

According to International Patent Classification (IPC) or to both national classification and IPC

B. FIELDS SEARCHED

Minimum documentation searched (classification system followed by classification symbols)
A61K51/00, C07D471/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), EMBASE(STN), MEDLINE(STN), REGISTRY(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br>A | LU Chun-Xiong et al., Synthesis and biodistribution of [$^{131}$I]IMPY, Nuclear Science and Techniques, 2005, Vol.16, No.5, p.289-292 | 6-10,17-28<br>1-5,11-16, |
| X<br>A | SUNDBERG, R.J. et al., Preparation of 2-Aryl and 2-Aryloxymethyl Imidazo[1,2-a]pyridines and Related Compounds, Journal of Heterocyclic Chemistry, 1988, Vol.25, No.1, p.129-137 | 23-28<br>1-22 |
| X<br>A | WO 2004/087641 A1 (Daiichi Pharmaceutical Co., Ltd.),<br>14 October, 2004 (14.10.04),<br>Referential examples 46, 170, 172, 175<br>& EP 1612204 A1 & US 2006/0276433 A1 | 23-28<br>1-22 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 November, 2008 (25.11.08) | 02 December, 2008 (02.12.08) |
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2008/069503

C (Continuation). DOCUMENTS CONSIDERED TO BE RELEVANT

| Category* | Citation of document, with indication, where appropriate, of the relevant passages | Relevant to claim No. |
|---|---|---|
| X<br>A | LANGE, J. et al., A structure-activity relationship study of the affinity of selected imidazo[1,2-a]pyridine derivatives, congeners of zolpidem, for the omega1-subtype of the benzodiazepine receptor, Acta Poloniae Pharmaceutica-Drug Research, 2001, Vol.58, No.1, p.43-52 | 23-28<br>1-22 |
| X<br>A | JP 2005-500315 A (SmithKline Beecham Corp.), 06 January, 2005 (06.01.05), Examples<br>& WO 2003/000689 A1 & EP 1401836 A1<br>& US 2005/0228004 A1 | 23-28<br>1-22 |
| X<br>A | JP 2005-508955 A (SmithKline Beecham Corp.), 07 April, 2005 (07.04.05), Examples<br>& WO 2003/031446 A1 & EP 1432712 A1<br>& US 2004/0248917 A1 | 23-28<br>1-22 |
| X<br>A | HERVET, M. et al., Comparative study on the reactivity of 6-haloimidazo[1,2-a]pyridine derivatives towards Negishi- and Stille-coupling reactions, Helvetica Chimica Acta, 2003, Vol.86, No.10, p.3461-3469 | 23-28<br>1-22 |
| X<br>A | WO 2005/043630 A1 (Idemitsu Kosan Co., Ltd.), 12 May, 2005 (12.05.05), Page 48<br>& EP 1679747 A1 & US 2007/0120111 A1 | 23-28<br>1-22 |
| X<br>A | WO 2005/086808 A2 (THE UNIVERSITY OF NORTH CAROLINA AT CHAPELHILL), 22 September, 2005 (22.09.05), Scheme 3 and 4<br>& EP 1745045 A2 & US 2005/0282853 A1 | 23-28<br>1-22 |
| X<br>A | Koubachi, J. et al., Synthesis of Polysubstituted Imidazo[1,2-a]pyridines via Microwave-Assisted One-Pot Cyclization/Suzuki Coupling/Palladium-Catalyzed Heteroarylation, Journal of Organic Chemistry, 2007.09, Vol.72, No.20, pp.7650-7655 | 23-28<br>1-22 |
| X<br>A | WO 2007/071434 A1 (SMITHKLINE BEECHAM CORP.), 28 June, 2007 (28.06.07), Intermediate 9, 83, 87<br>(Family: none) | 23-28<br>1-22 |
| X<br>A | Gudmundsson, K.S. et al., Imidazo[1,2-a] pyridines with potent activity against herpesviruses, Bioorganic & Medicinal Chemistry Letters, 2007.05, Vol.17, No.10, 2735-2739 | 23-28<br>1-22 |

Form PCT/ISA/210 (continuation of second sheet) (April 2007)

**INTERNATIONAL SEARCH REPORT**

International application No.
PCT/JP2008/069503

C (Continuation). DOCUMENTS CONSIDERED TO BE RELEVANT

| Category* | Citation of document, with indication, where appropriate, of the relevant passages | Relevant to claim No. |
|---|---|---|
| X<br>A | KATSIFIS, A. et al., Synthesis of [$^{123}$I]iodine labelled imidazo[1,2-b]pyridazines as potential probes for the study of peripheral benzodiazepine receptors using SPECT, Radiochimica Acta, 2004, Vol.92, No.4-6, p.305-309 | 23,24,26,27<br>1-22,25,28 |
| X<br>A | BARLIN, G.B., Imidazo[1,2-b]pyridazines: Syntheses and interaction with central and peripheral-type (mitochondrial) benzodiazepine receptors, Journal of Heterocyclic Chemistry, 1998, Vol.35, No.5, p.1205-1217 | 23,24,26,27<br>1-22,25,28 |
| X<br>A | WO 2007/110438 A1 (FERRER INTERNATIONAL, S.A.),<br>04 October, 2007 (04.10.07),<br>Page 24<br>& EP 1845098 A1 | 23,24,26,27<br>1-22,25,28 |
| X<br>A | WO 2007/028051 A1 (ABBOTT LABORATORIES),<br>08 March, 2007 (08.03.07),<br>Pages 48, 50, 51<br>& US 2007/0099925 A1 | 23,24,26,27<br>1-22,25,28 |
| X<br>A | JP 2004-525192 A (F. Hoffmann-La Roche AG.),<br>19 August, 2004 (19.08.04),<br>Examples<br>& WO 2002/092086 A1 & EP 1381363 A1<br>& US 2002/0188128 A1 | 23-25<br>1-21,26-28 |
| X<br>A | KATSIFIS, A. et al., Synthesis of [$^{123}$I]N',N'-Dimethyl-6-methyl-(4'-iodophenyl)imidazo[1,2-a]pyridine-3-acetamide for the study of peripheral benzodiazepine receptors using SPECT, Journal of Labelled Compounds and Radiopharmaceuticals, 2000, Vol.43, No.4, p.385-394 | 23-25<br>1-21,26-28 |
| X<br>A | Kim, M.K. et al., 3D-QSAR of PET agents for imaging β-amyloid in Alzheimer's disease, Bulletin of the Korean Chemical Society, 2007.06, Vol.28, No.7, pp.1231-1234 | 23-25<br>1-21,26-28 |
| X<br>A | WO 2007/034282 A2 (PFIZER PRODUCTS INC.),<br>29 March, 2007 (29.03.07),<br>Preparation 28<br>(Family: none) | 23-25<br>1-21,26-28 |
| X<br>A | WO 2007/019416 A1 (SIRTRIS PHARMACEUTICALS, INC.),<br>15 February, 2007 (15.02.07),<br>Page 347<br>& US 2007/0037809 A1 & EP 1853610 A | 23-25<br>1-21,26-28 |

Form PCT/ISA/210 (continuation of second sheet) (April 2007)

**INTERNATIONAL SEARCH REPORT**

International application No.
PCT/JP2008/069503

C (Continuation). DOCUMENTS CONSIDERED TO BE RELEVANT

| Category* | Citation of document, with indication, where appropriate, of the relevant passages | Relevant to claim No. |
|---|---|---|
| X<br>A | Yadav, J.S. et al., Cu(OTf)2-catalyzed synthesis of imidazo[1,2-a]pyridines from α-diazoketones and 2-aminopyridines, Tetrahedron Letters, 2007.10.22, Vol.48, No.43, pp.7717-7720 | 23-25<br>1-21,26-28 |
| X<br>A | BARLIN, G. B. et al., Imidazo[1,2-b]pyridazines. XX Syntheses of some 3-acylaminomethyl-6-(chloro, fluoro, methoxy, methylthio, phenoxy and phenylthio)-2-(phenyl, 4-t-butylphenyl, 4-cyclohexylphenyl, beta-naphthyl and styryl) imidazo[1,2-b]pyridazines and their interaction with central and peripheral-type benzodiazepine receptors, Australian Journal of Chemistry, 1996, Vol.49, No.4, p.451-461 | 26-28<br>1-25 |
| X<br>A | WO 2007/063946 A1 (Daiichi Radioisotope Laboratories, Ltd.), 07 June, 2007 (07.06.07), Referential examples 1, 13, 28 (Family: none) | 26-28<br>1-25 |
| X<br>A | US 2007/0117804 A1 (Lianyun Zhao), 24 May, 2007 (24.05.07), Example 171 & WO 2007/058942 A2 | 26-28<br>1-25 |
| X<br>A | Danqian XU et al., Synthesis of 2-arylimidazo [1,2-a]pyrimidines by the Chichibabin synthesis in ionic liquids, Journal of Chemical Research, Synopses, 2003, No.10, p.645-647 | 23,24<br>1-22,25-28 |
| P,X | WO 2008/059714 A1 (Nihon Medi-Physics Co., Ltd.), 22 May, 2008 (22.05.08), Full text (Family: none) | 1-28 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007002540 A
- WO 2007063946 A
- JP 2004506723 T
- JP 2005504055 T
- JP 2005512945 T
- JP 2002523383 T
- WO 2005016888 A
- WO 03106439 A


### Non-patent literature cited in the description


- **J. A. Hardy ; G. A. Higgins.** Alzheimer's Disease: The Amyloid Cascade Hypotheses. *Science,* 1992, vol. 256, 184-185
- **G. McKhann et al.** clinical diagnosis of Alzheimer's disease: Retort of the NINCDS-ADRDA Work Group under the auspices of Department of Health and Human Services Task Force on Alzheimer's Disease. *Necrology,* 1984, vol. 34, 939-944
- **Z.-P. Zhuang et al.** Radioiodinated Styrylbenzenes and Thioflavins as Probes for Amyloid Aggregates. *J. Med. Chum.,* 2001, vol. 44, 1905-1914
- **Masahiro One et al.** 11C-labeled stilbene derivatives as Aβ-aggregate-specific PET imaging agents for Alzheimer's disease. *Nuclear Medicine and Biology,* 2003, vol. 30, 565-571
- **H. F. Kung et al.** Novel Stilbenes as Probes for amyloid plaques. *J. American Chemical Society,* 2001, vol. 123, 12740-12741
- **Zhi-Ping Zhuang et al.** IBOX(2-(4 -dimethylaminophenyl)-6-iodobensoxazole) : a ligand for imaging amyloid plaques in the brain. *Nuclear medicine and Biology,* 2001, vol. 28, 887-894
- **Furumoto Y et al.** [11C]BF-227: A New C-Labeled 2-Ethenylbenzoxazole Derivative for Amyloid-β Plaques Imaging. *European Journal of Nuclear Medicine and Molecular Imaging,* 2005, vol. 32 (1), 759
- **Eric D. Agdeppa et al.** Dialkylamino-6-Acylmalononitrile Substituted Naphthalenes (DDNP Analogs): Novel Diagnostic and Therapeatic Tools in Alzheimer's Disease. *Molecular Imaging and Biology,* 2003, vol. 5, 404-417
- **Zhi-Ping Zhuang et al.** Structure-Activity. Relationship of Imidazo[1,2-a]pyridines as Ligands for Detecting β-Amyloid Plaques in the Brain. *J. Med. Chem,* 2003, vol. 46, 237-243
- **W. E. Klunk et al.** Imaging brain amyloid in Alzheimer's disease with Pittsburgh Compound-B. *Ann. Neurol.,* 2004, vol. 55, 306-319
- **Nicolaas P. L. G. Verhoeff et al.** In-Vivo Imaging of Alzheimer Disease β-Amyloid with [11C]SB-13 PET. *American Journal of Geriatric Psychiatry,* 2004, vol. 12, 584-595
- **Hiroyuki Arai et al.** [11C]-BF-227 AND PET to visualize Amyloid in Alzheimer's Disease Patients. *Alzheimer's & Dementia: The Journal of the Alzheimer's Association,* 2006, vol. 2 (1), 312
- **Christopher M. Clark et al.** Imaging Amyloid with I123 IMPY SPECT. *Alzheimer's & Dementia: The Journal of the Alzheimer's Association,* 2006, vol. 2 (1), 342
- **D. M. Skovronsky et al.** *Proc. Natl. Acad. Sci.,* 2000, vol. 97, 7609
- **Zhi-Ping Zhuang et al.** *Nuclear Medicine and Biology,* 2001, vol. 28, 887-894
- **H. F. Kung et al.** *J. Am. Chem. Soc.,* 2001, vol. 123, 12740-12741
- **Masahiro Onto et al.** *Nuclear Medicine and Biology,* 2003, vol. 30, 565-571
- **Joseph G. Lombardino.** *Journal of Medical Chemistry,* 1981, vol. 24, 39-42
- **King, L. Carroll ; Ostrum, G. Kenneth.** *Journal of Organic Chemistry,* 1964, vol. 29 (12), 3459-3461
- **Zhi-Ping Zhuang et al.** *J. Med. Chem,* 2003, vol. 46, 237-243
- **Naiki, H. et al.** *Laboratory Investigation,* 1996, vol. 74, 374-383
- **Douglas D. Dischino et al.** *J. Nucl. Med.,* 1983, vol. 24, 1030-1038